(19) Europäisches Patentamt
European Patent Office
Office européen des brevets

(11) Publication number: 0 207 454 B1

(12) **EUROPEAN PATENT SPECIFICATION**

(45) Date of publication of patent specification: **09.09.92**

(51) Int. Cl.5: **C07D 413/12**, C07D 413/10, A61K 31/42, A61K 31/535, C07D 261/08, //C07C233/21, C07D263/14,C07C205/03, C07D307/42,C07D307/46, C07D263/32,C07D263/34, C07C47/56

(21) Application number: **86108745.0**

(22) Date of filing: **26.06.86**

(54) **Isoxazole and furan derivatives, their preparation and use as antiviral agents.**

(30) Priority: **02.07.85 US 751348**

(43) Date of publication of application:
**07.01.87 Bulletin 87/02**

(45) Publication of the grant of the patent:
**09.09.92 Bulletin 92/37**

(84) Designated Contracting States:
**AT BE CH DE FR GB IT LI NL SE**

(56) References cited:
**EP-A- 0 111 345**
**EP-A- 0 137 242**

(73) Proprietor: **STERLING WINTHROP INC.**
**90 Park Avenue**
**New York, NY 10016(US)**

(72) Inventor: **Diana, Guy Dominic**
**Box 192 Garfield Road**
**East Nassau New York(US)**
Inventor: **Carabateas, Philip Michael**
**Box 432 R.D. 1**
**Nassau New York(US)**

(74) Representative: **Baillie, Iain Cameron et al**
**c/o Ladas & Parry, Altheimer Eck 2**
**W-8000 München 2(DE)**

**EP 0 207 454 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to isoxazoles and furans having a phenyl-aliphatic side chain in which the phenyl group is further substituted by an oxazole or 1,3-oxazine moiety.

Sterling Drug Inc. European Patent Application Publ. No. 137,242, published April 17, 1985, discloses antivirally active compounds having the formula

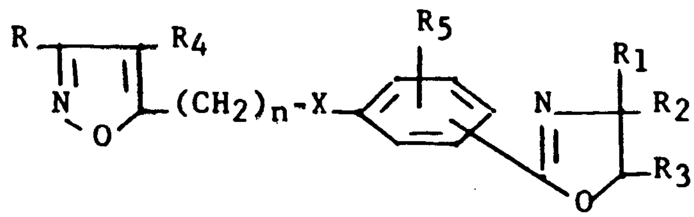

wherein:

R, $R_1$, $R_2$, $R_3$ and $R_4$ are each hydrogen or alkyl of 1 to 3 carbon atoms optionally substituted by hydroxy, lower-alkanoyloxy, lower-alkoxy, chloro, or N=Z, wherein N=Z is amino, lower-alkanoylamino, lower-alkylamino, di-lower-alkylamino, 1-pyrrolidinyl, 1-piperidinyl or 4-morpholinyl; with the proviso that R is other than hydrogen;

$R_5$ is hydrogen, lower-alkyl, halogen, nitro, lower-alkoxy, lower-alkylthio or trifluoromethyl;

X is 0 or a single bond; and

n is an integer from 3 to 9;

and to pharmaceutically acceptable acid-addition salts thereof.

It has now been found that related compounds wherein the phenyl ring is substituted in both positions adjacent to the isoxazole-bearing side chain by selected substituents generally have improved properties as compared with the corresponding mono-substituted or unsubstituted compounds.

Accordingly, the present invention relates to compounds of the formula

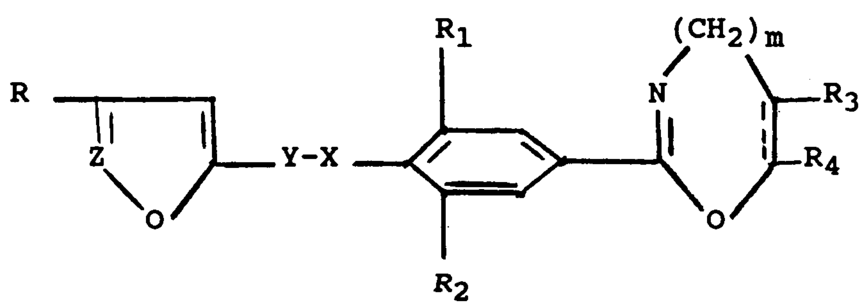

I

wherein:

R is hydrogen or alkyl of 1 to 3 carbon atoms optionally substituted by hydrory or lower alkoxy of 1-4 carbon atoms;

X is 0 or $CH_2$;

Y is an alkylene bridge of 3-9 carbon atoms of an olefinic linkage of 5 carbon atoms with one unsaturated bond;

Z is N or $R_5C$, where $R_5$ is hydrogen or lower alkanoyl of 1-4 carbon atoms, with the proviso that when Z is N, R is other than hydrogen;

m = 0 or 1;

$R_1$ and $R_2$ are each halogen, methyl, nitro, or trifluoromethyl; and

$R_3$ and $R_4$ are hydrogen or lower-alkyl of 1-3 carbon atoms;

and to pharmaceutically acceptable acid-addition salts thereof.

The dashed line in the oxazole or 1,3-oxazine ring indicates that a double bond is optionally present in the position indicated.

A preferred class of compounds within the scope of Formula **I** are those of the formula

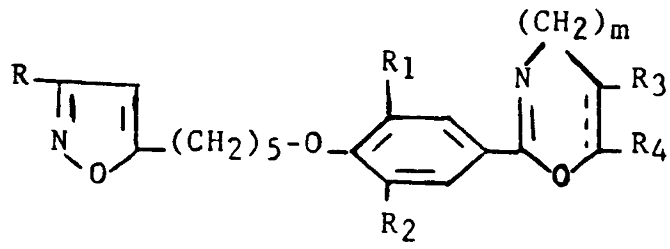

II

wherein:

m = 0 or 1;

R is methyl or hydroxymethyl;

$R_1$ and $R_2$ are halogen, methyl, nitro, or trifluoromethyl; and

$R_3$ and $R_4$ are hydrogen or lower-alkyl of 1-3 carbon atoms;

and to pharmaceutically acceptable acid-addition salts thereof.

A preferred species within the scope of Formula **II** is 5-{5-[2,6-dichloro-4-(4,5-dihydro-2-oxazolyl)-phenoxy]pentyl}isoxazole [m = 0, R = $CH_3$, $R_1$ and $R_2$ = Cl, $R_3$ and $R_4$ = H].

Compositions for combating viruses comprise an antivirally effective amount of a compound of Formulas **I** or **II** in admixture with a suitable carrier or diluent.

One can prepare compounds within the scope of Formula I where X is 0 by reacting a compound of the formula

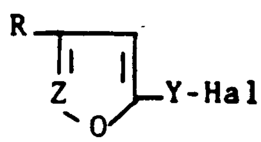

wherein Hal is chlorine, bromine or iodine, with an alkali metal salt of a compound of the formula

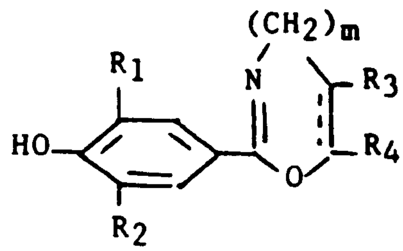

One can also prepare a compound of Formula I but not having a double bond in the 4,5-position of the oxazole or oxazine ring and wherein R is hydrogen or lower alkyl of 1 to 3 carbon atoms by cyclizing a compound of the formula

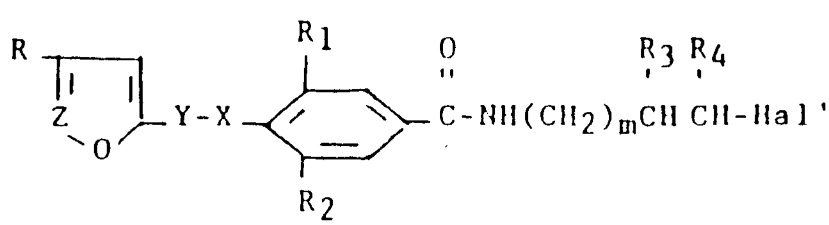

III

wherein Hal' is chlorine or bromine, by heating the latter in an inert solvent in the presence of an acid-acceptor.

Also disclosed are novel, antivirally active, intermediates of Formula **III**.

The compounds of Formulas **I** and **II** are sufficiently basic to form stable acid-addition salts with strong acids, and said salts are within the purview of the invention. The nature of the acid-addition salt is immaterial, provided it is derived from an acid the anion of which is essentially non-toxic to animal organisms. Examples of appropriate acid-addition salts include the hydrochlorIde, hydrobromide, sulfate, acid sulfate, maleate, citrate, tartrate, methanesulfonate, p-toluenesulfonate, dodecyl sulfate, and cyclohexanesulfamate.

Throughout this specification and claims when the terms "lower alkyl, "lower alkoyl" or "lower alkanoyl" are used, they refer to such groups containing from one to four carbon atoms.

Throughout this specification, when the term halogen is used to define the substituents $R_1$ and $R_2$, any of the four common halogens, fluorine, chlorine, bromine or iodine are contemplated, with chlorine and bromine being preferred.

The process of preparing compounds of Formula **I** wherein X is 0 by reacting a compound of the formula

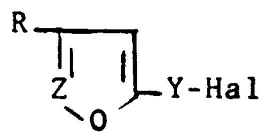

**IV**

with a compound of the formula

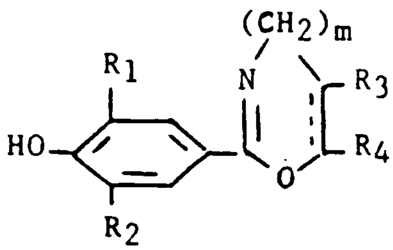

**V**

is carried out by heating the reactants in an inert solvent in the presence of an alkali metal base, e.g. potassium carbonate, at a temperature between about 50° and 150° C.

The intermediates of Formula **IV** where Z is N are prepared by reacting an alkali metal derivative of an isoxazole of the formula

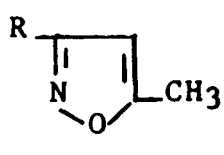

**VI**

wherein R is alkyl or hydroxyalkyl of 1 to 3 carbon atoms with a dihalide, Hal-Y'-Hal, where Y' is an alkylene bridge of 2 to 8 carbon atoms optionally interrupted by an olefinic linkage. Said alkali metal derivative is prepared in situ by treating the compound of Formula **VI** with an organo-alkali metal base under anhydrous conditions. Preferred organo-alkali metal bases are butyllithium and lithium diisopropylamide.

The intermediates of Formula **IV** where Z is $R_8C$ are prepared from the appropriate omega-(2-furan)-alkanoic acid by reduction to the corresponding alcohol and replacement of the hydroxy group by halogen; or by direct alkylation of furan with a dihalide, Hal-Y-Hal, in the presence of a strong base such as butyllithium.

The intermediates of Formula **VI** are a generically known class of oxazole substituted phenols, prepared as described hereinafter in the general description and specific examples.

The following flow sheet sets forth the preferred synthetic methods for preparing 4,5-dihydrooxazoles of the invention.

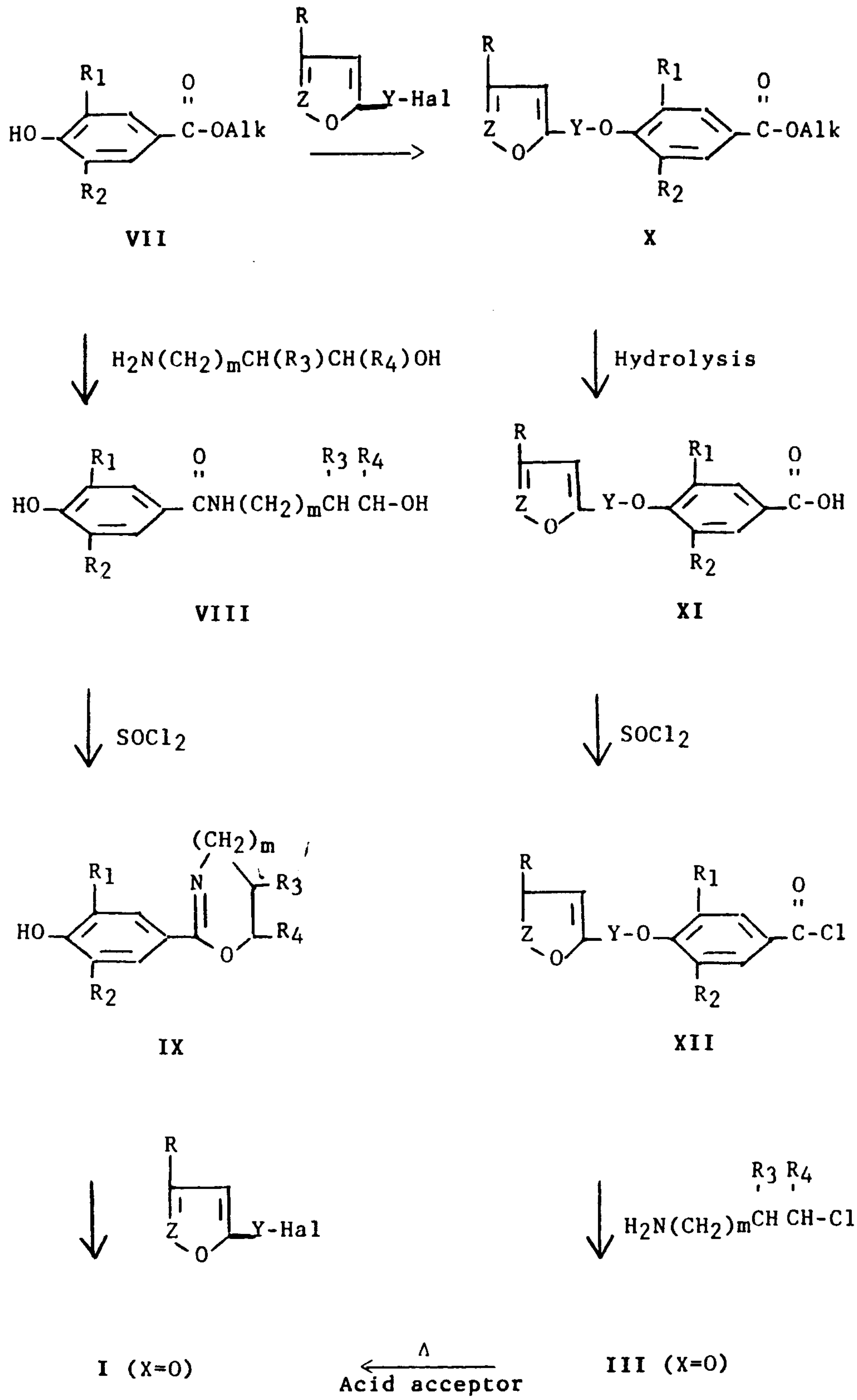


A hydroxybenzoate (**VII**, Alk = lower-alkyl) is condensed with a hydroxyalkylamine to give a hydroxyalkylamide (**VIII**). The latter is then cyclized with thionyl chloride to give a substituted phenol of Formula **IX**. The final step is the reaction of the phenol with a haloalkylisoxazole or -furan as described above.

Alternatively, the hydroxybenzoate (**VII**) is caused to react with a halo-alkylisoxazole or -furan to give the ester (X) which is hydrolyzed to the corresponding acid (**XI**). The latter is then converted to its acid

chloride (**XII**) which is reacted with a chloroalkylamine to give a chloroalkylamide intermediate of Formula **III** (X = 0). The latter when heated in the presence of an acid-acceptor is converted to a final product of Formula **I** (X = 0). The latter cyclization takes place in an inert solvent at a temperature between 50 ° C and 150 ° C., conveniently at the reflux temperature of the solvent mixture. The acid-acceptor can be any basic substance capable of absorbing the hydrogen chloride produced in the reaction while otherwise inert in the reaction. Such acid-acceptors may be tertiary-amines or inorganic bases such as potassium carbonate. A preferred acid-acceptor is 1,8-diazabicyclo[5.4.0]undec-7-ene.

Variations in the sequence of steps **VII** → **X** → **XI** can be employed using compounds with nitrile or formyl groups in place of the ester moiety, with eventual conversion to the free carboxyl group.

The compounds of Formulas **I** and **II** having a double bond in the 4,5-position of the oxazole ring are prepared by a procedure analogous to that described above, namely by reaction of a phenol of formula

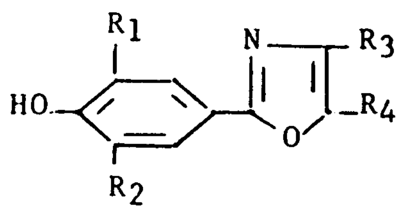

**XIII**

with a compound of Formula **IV**. The intermediates of Formula **XIII** are in turn prepared by reacting an aldehyde of the formula

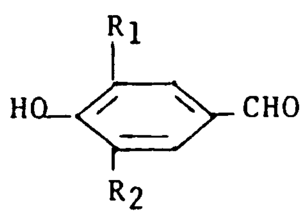

with an oxime of the formula $R_3C(=NOH)\text{-}COR_4$ and reducing the resulting oxazole N-oxide with zinc or titanium trichloride.

Compounds of Formula **I** where X is $CH_2$ are prepared by alkylation of a compound of the formula

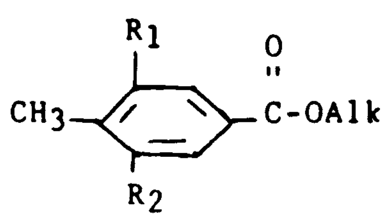

with a haloalkylisoxazole or -furan of Formula **IV** to give an ester of the formula

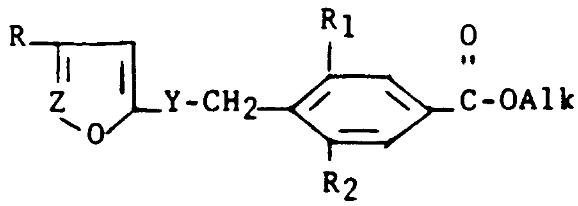

XIV

The ester moiety of Formula **XIV** is then manipulated by procedures analogous to the reaction sequence **X** → **XI** → **XII** → **III** → **I** to form the oxazole or oxazine ring.

The compounds of Formula **I** where R is lower-alkyl substituted by methoxy are most conveniently prepared from the corresponding compounds of Formula I where R is hydroxyalkyl.

Etherification of a hydroxyalkyl compound by conventional procedures, as by reaction with a lower-alkyl halide in the presence of a strong base, gives the corresponding lower-alkoxy derivative.

A hydroxyalkyl compound can be converted to a haloalkyl compound by reaction with a reagent such as thionyl halide or phosphorus trihalide, capable of replacing aliphatic hydroxy groups by halogen.

The haloalkyl compounds are in turn convertible to aminoalkyl compounds by reaction with ammonia or an amine, HN=Z'. Compounds where HN=Z' is lower-alkanoylamino are prepared by acylation of the compounds where HN=Z' is $NH_2$ with a lower-alkanoyl halide or anhydride, lower-alkanoyl preferably having from 1 to 4 carbon atoms.

The structures of the compounds of the invention were established by the modes of synthesis, by elementary analysis, and by infrared and nuclear magnetic resonance spectra.

The following examples will further illustrate the invention.

Example 1

a) 3,5-Dichloro-4-hydroxy-N-(2-hydroxyethyl)benzamide [**VIII**; $R_1$ and $R_2$ = Cl, $R_3$ and $R_4$ = H, m = 0].

A 2 L, 3 necked, round bottom flask was charged with 2-aminoethanol (240 gm; 3.93 moles) and heated to 80 °C. Methyl 3,5-dichloro-4-hydroxybenzoate (432 gm; 1.96 moles) was added in portions through a powder funnel. The resulting amber solution was heated to 145 °C and the liberated methanol distilled into a Dean Stark trap. The reaction requires about 3.5 hrs. Upon completion, the solution was cooled to 90-100 °C and dissolved in 1.95 L $H_2O$. The aqueous solution was cooled to 25 °C, placed in an ice bath and made slightly acidic with concentrated hydrochloric acid (196 ml; 2.35 moles). The product precipitated and filtration afforded a white solid. After drying at 50 °C in a vacuum oven, the product darkened slightly. The crude material (384.4 gm; 78.8%) was ground and passed through a No. 20 mesh screen to produce 3,5-dichloro-4-hydroxy-N-(2-hydroxyethyl)benzamide as a cream colored solid (m.p. 174-178 °C) which is suitable for use in the next step.

b) 2,6-Dichloro-4-(4,5-dihydro-2-oxazolyl)phenol [**IX**; $R_1$ and $R_2$ = Cl, $R_3$ and $R_4$ = H, m = 0].

3,5-Dichloro-4-hydroxy-N-(2-hydroxyethyl)benzamide (400 gm, 1.61 moles) was ground and sifted through a No. 20 mesh screen prior to use. To a stirred suspension of the above in 2.8 L isopropyl acetate was added thionyl chloride (285 gm, 2.41 moles) in a steady stream. An exotherm to 45-50 °C developed and the gray suspension appeared lighter after a short time. After stirring 2.5 hr, the suspension was cooled to room temperature and filtered. The cake was rinsed with isopropyl acetate and dried in a vacuum oven at room temperature overnight. There was obtained 371 gm (86% yield) of 2,6-dichloro-4-(4,5-dihydro-2-oxazolyl)phenol in the form of its hydrochloride salt, m.p. 189-191 °C, acceptable for use in the next step. The purified free base had the m.p. 195 °C (decompn.).

c) 5-[5-[2,6-Dichloro-4-(4,5-dihydro-2-oxazolyl)phenoxy]pentyl]-3-methylisoxazole [**II**; R = $CH_3$, $R_1$ and $R_2$ = Cl, $R_3$ and $R_4$ = H, m = 0].

To a stirred suspension of milled potassium carbonate (172.5 gm, 1.25 moles) in 1.35 L dimethylformamide (DMF) was added 2,6-dichloro-4-(4,5-dihydro-2-oxazolyl)phenol hydrochloride (133.5 gm, 0.5 moles). Heavy gas evolution ($CO_2$) occurred and the reaction was warmed on the steam bath to 70 °C. After stirring for about 5 minutes, 5-(5-bromopentyl)-3-methylisoxazole (121.8 gm, 0.525 moles) was added in one portion. The mixture was heated to 90-95 °C for 1 hr, allowed to cool to room temperature and then filtered. The filter cake was rinsed with DMF and the filtrate was concentrated under water vacuum to a viscous brown oil (260 gm). This residue was dissolved in 300 ml isopropyl acetate and

washed with water and then with brine. The organic layer was dried over $MgSO_4$, charcoaled, filtered and evaporated to dryness. The crude product (220 gm oil) was diluted with 287 ml acetone (1.5 volumes based on theoretical yield) and was cooled and stirred in a Dry Ice/acetone bath to -25°C. The product was collected and air dried to give 115 gm (60% yield) of 5-{5-[2,6-dichloro-4-(4,5-dihydro-2-oxazolyl)-phenoxylpentyl}-3-methylisoxazole, m.p. 38-40°C. A sample when recrystallized from triethylamine had the m.p. 42-43°C.

5-{5-[2,6-Dichloro-4-(4,5-dihydro-2-oxazolyl)phenoxy]pentyl}-3-methylisoxazole when treated with methanesulfonic acid formed an acid-addition salt with m.p 98-101°C.

The intermediate 5-(5-bromopentyl)-3-methylisoxazole was prepared from 1,4-dibromobutane and the lithium salt of 3,5-dimethylisoxazole produced in situ with n-butyllithium and diisopropylamine in tetrahydrofuran solution.

By the processes described above in Example 1 were prepared the following compounds:

Example 2

5-{5-[4-(4,5-Dihydro-2-oxazolyl)-2,6-dimethylphenoxylpentyl}-3-methylisoxazole [**II**; R, $R_1$ and $R_2$ = $CH_3$, $R_3$ and $R_4$ = H, m = 0], amber oil by chromatography, m.p. below room temperature; monomethanesulfonate salt, m.p. 114-115°C.

Intermediates: 5-(5-bromopentyl)-3-methylisoxazole and 4-(4,5-dihydro-2-oxazolyl)-2,6-dimethylphenol, hydrochloride salt, m.p. 208-210°C (from methanol).

Example 3

5-{5-[2,6-Dibromo-4-(4,5-dihydro-2-oxazolyl)phenoxy]pentyl}-3-methylisoxazole [**II**; R = $CH_3$, $R_1$ and $R_2$ = Br, $R_3$ and $R_4$ = H, m = 0], light yellow oil by chromatography, m.p. below room temperature.

Intermediates: 5-(5-bromopentyl)-3-methylisoxazole and 2,6-dibromo-4-(4,5-dihydro-2-oxazolyl)phenol hydrochloride, in turn prepared by cyclization of 3,5-dibromo-4-hydroxy-N-(2-hydroxyethyl)benzamide [**VIII**; $R_1$ and $R_2$ = Br, $R_3$ and $R_4$ = H, m = 0], m.p. 172-173°C.

Example 4

5-{6-[2,6-Dichloro-4-(4,5-dihydro-2-oxazolyl)phenoxy]hexyl}-3-methylisoxazole [**I**; Y = $(CH_2)_6$, X = O, Z = N, m = 0, R = $CH_3$, $R_1$ and $R_2$ = Cl, $R_3$ and $R_4$ = H], m.p. 48-50°C (from triethylamine).

Intermediates: 5-(6-bromohexyl)-3-methylisoxazole and 2,6-dichloro-4-(4,5-dihydro-2-oxazolyl)phenol (Example 1b).

Example 5

5-{5-[2,6-Dichloro-4-(4,5-dihydro-4-methyl-2-oxazolyl)phenoxy]pentyl}-3-methylisoxazole [**II**; R = $CH_3$, $R_1$ and $R_2$ = Cl, $R_3$ = $CH_3$, $R_4$ = H, m = 0], viscous yellow oil by chromatography, m.p. below room temperature.

Intermediates: 5-(5-bromopentyl)-3-methylisoxazole and 2,6-dichloro-4-(4,5-dihydro-4-methyl-2-oxazolyl)phenol, m.p. 145-146°C, in turn prepared from 3,5-dichloro-4-hydroxy-N-(2-hydroxy-1-methylethyl)-benzamide, m.p. 168-170°C (from acetonitrile).

Example 6

5-{5-[2,6-Dichloro-4-(4,5-dihydro-5-methyl-2-oxazolyl)phenoxy]pentyl}-3-methylisoxazole [**II**; R = $CH_3$, $R_1$ and $R_2$ = Cl, $R_3$ = H, $R_4$ = $CH_3$, m = 0], yellow oil by chromatography, m.p. below room temperature.

Intermediates: 5-(5-bromopentyl)-3-methylisoxazole and 2,6-dichloro-4-(4,5-dihydro-5-methyl-2-oxazolyl)phenol, m.p. 173-174°C, in turn prepared from 3,5-dichloro-4-hydroxy-N-(2-hydroxypropyl)-benzamide, m.p. 126-128°C (from isopropyl acetate).

Example 7

5-{5-[2,6-Dichloro-4-(4,5-dihydro-2-oxazolyl)phenoxy]hexyl}-3-methylisoxazole [**I**; Y = $CH_2CH_2CH_2CH_2CH(CH_3)$, X = O, Z = N, m = 0, R = $CH_3$, $R_1$ and $R_2$ = Cl, $R_3$ and $R_4$ = H], colorless oil by chromatography.

Intermediates: 5-(5-bromohexyl)-3-methylisoxazole and 2,6-dichloro-4-(4,5-dihydro-2-oxazolyl)phenol.

Example 8

5-{4-[2,6-Dichloro-4-(4,5-dihydro-2-oxazolyl)phenoxy]pentyl}-3-methylisoxazole [**I**; Y = $CH_2CH_2CH_2CH(CH3)$, X = O, Z = N, m = 0, R = $CH_3$, $R_1$ and $R_2$ = Cl, $R_3$ and $R_4$ = H], yellow oil by chromatography.

Intermediates: 5-(4-bromopentyl)-3-methylisoxazole and 2,6-dichloro-4-(4,5-dihydro-2-oxazolyl)phenol.

Example 9

5-{5-[2-Bromo-4-(4,5-dihydro-2-oxazolyl)-6-nitrophenoxy]pentyl}-3-methylisoxazole [**II**; R = $CH_3$, $R_1$ = Br, $R_2$ = $NO_2$, $R_3$ and $R_4$ = H, m = 0], yellow oil by chromatography.

Intermediates: 5-(5-bromopentyl)-3-methylisoxazole and 2-bromo-4-(4,5-dihydro-2-oxazolyl)-6-nitrophenol. The latter, a bright yellow solid, was prepared by cyclization of 3-bromo-4-hydroxy-N-(2-hydroxyethyl)-5-nitrobenzamide,yellow solid, m.p. 163-164 ° C (from methanol-isopropyl acetate). The latter was in turn prepared by bromination of 4-hydroxy-3-nitrobenzoic acid to form 5-bromo-4-hydroxy-3-nitrobenzoic acid, m.p. 233-234 ° C (yellow solid from isopropyl acetate), esterification to the corresponding methyl ester, m.p. 128-130 ° C (yellow solid from carbon tetrachloride) and reaction of the latter with 2-hydroxyethylamine.

Example 10

5-{5-[2,6-Dichloro-4-(4,5-dihydro-2-oxazolyl)phenoxy]-3-pentenyl}-3-methylisoxazole (Z-isomer) [**I**; Y = $(CH_2)_2CH$=-$CHCH_2$, X = O, Z = N, m = 0, R = $CH_3$, $R_1$ and $R_2$ = Cl, $R_3$ and $R_4$ = H], colorless solid, m.p. 52-54 ° C (from t-butyl methyl ether).

Intermediates: 2,6-dichloro-4-(4,5-dihydro-2-oxazolyl)phenol and the isomer of 5-(5-chloro-3-pentenyl)-3-methylisoxazole prepared from cis-1,4-dichloro-2-butene and 3,5-dimethylisoxazole.

Example 11

5-{5-[2,6-Dichloro-4-(4,5-dihydro-2-oxazolyl)phenoxy]-3-pentenyl}-3-methylisoxazole (E-isomer) [**I**; Y = $(CH_2)_2CH=CHCH_2$, X = O, Z = N, m = 0, R = $CH_3$, $R_1$ and $R_2$ = Cl, $R_3$ and $R_4$ = H], colorless solid, m.p. 59-61 ° C (from ether-hexane).

Intermediates: 2,6-dichloro-4-(4,5-dihydro-2-oxazolyl)phenol and the isomer of 5-(5-chloro-3-pentenyl)-3-methylisoxazole prepared from trans-1,4-dichloro-2-butene and 3,5-dimethylisoxazole.

Example 12

5-{7-[2,6-Dichloro-4-(4,5-dihydro-2-oxazolyl)phenoxy]heptyl}-3-methylisoxazole [**I**; Y = $(CH_2)_7$, X = O, Z = N, m = 0, R = $CH_3$, $R_1$ and $R_2$ = Cl, $R_3$ and $R_4$ = H], colorless solid, m.p. 36-37 ° C (from hexane-ether).

Intermediates: 5-(7-bromoheptyl)-3-methylisoxazole and 2,6-dichoro-4-(4,5-dihydro-2-oxazolyl)phenol.

Example 13

5-{3-[2,6-Dichloro-4-(4,5-dihydro-2-oxazolyl)phenoxy]propyl}-3-methylisoxazole [**I**; Y = $(CH_2)_3$, X = O, Z = N, m = 0, R = $CH_3$, $R_1$ and $R_2$ = Cl, $R_3$ and $R_4$ = H], colorless solid, m.p. 67-68 ° C (from ether).

Intermediates: 5-(3-iodopropyl)-3-methylisoxazole and 2,6-dichloro-4-(4,5-dihydro-2-oxazolyl)phenol.

Example 14

2-{3,5-Dichloro-4-[5-(2-furanyl)penty]oxy]phenyl}-4,5-dihydrooxazole [**I**; Y = $(CH_2)_5$, X = O, Z = HC, m = 0, R = H, $R_1$ and $R_2$ = Cl, $R_3$ and $R_4$ = H], amber oil by chromatography.

Intermediates: 2,6-dichloro-4-(4,5-dihydro-2-oxazolyl)phenol and 2-(5-bromopentyl)furan, prepared from furan and 1,5-dibromopentane.

Example 15

a) 4-(2-Furanyl)-1,4-butadiene-1,1-dicarboxylic acid.

A mixture of 122.1 g of 2-furanacrolein, 225 g of malonic acid, 330 ml of pyridine and 0.5 ml of piperidine was stirred on a steam bath for 4 hours. The reaction mixture was concentrated in vacuo, 1 liter of water added, and the mixture acidified with 6N hydrochloric acid. The yellow solid product (120 g) was collected, washed with water, dried and used directly in the next reaction.

b) 5-(2-Furany])pentanoic acid.

The acid from part (a) was dissolved in aqueous potassium hydroxide and hydrogenated in the presence of 2 g of palladium-on-carbon catalyst for 4 hours. The hydrogenated product was acidified, isolated and refluxed with 150 ml of pyridine for 11 hours to cause decarboxylation. The resulting product was isolated and distilled to give 60.4 g of 5-(2-furanyl)pentanoic acid, b.p. 105-120 ° C(0.07 mm).

c) 5-(2-Furanyl)pentanol.

5-(2-Furanyl)pentanoic acid (60.4 g) was reduced with 13.7 g of lithium aluminum hydride in 400 ml of tetrahydrofuran, heated at reflux for about 16 hours. The product was isolated and purified by distillation to give 51.4 g of 5-(2-furanyl)pentanol, b.p. 70-73 ° C(0.1 mm).

d) 5-(2-Furanyl)pentanol acetate.

5-(2-Furanyl)pentanol (81.8 g) was esterified with 100 ml of acetic anhydride and 200 mg of dimethylaminopyridine to give 97.0 g of the acetate ester, b.p. 74-75 ° C(0.1 mm).

e) 5-(6-Acetyl-2-furanyl)pentanol acetate.

To a mixture of 100 ml of trifluoroacetic anhydride and 100 ml of glacial acetic acid was added 89 g of 5-(2-furanyl)pentanol acetate in 100 ml of acetic acid. The reaction mixture was allowed to stand at room temperature for about 16 hours, then poured into water and extracted with methylene dichloride. The extracts were concentrated and the residue distilled to give 60.6 g of 5-(6-acetyl-2-furanyl)pentanol acetate, b.p. 120-123 ° C (0.05 mm).

f) 5-(6-Acetyl-2-furanyl)pentanol.

5-(6-Acetyl-2-furanyl)pentanol acetate (60.6 g) was hydrolyzed with 3 g of sodium methoxide in 900 ml of absolute methanol and the product isolated to give 37.3 g of 5-(6-acetyl-2-furanyl)pentanol, b.p. 132-135 ° C(0.1 mm).

g) 2-(5-Chloropentyl)-6-acetylfuran [**IV**; Z = $CH_3COC$, Hal = Cl, R = H, Y = $(CH_2)_5$].

5-(6-Acetyl-2-furanyl)pentanol (9.8 g), 13.1 g of triphenylphosphine and 100 ml of carbon tetrachloride were combined and heated at reflux for 4 hours. The reaction mixture was concentrated in vacuo, and the residue was dissolved in ether and filtered. The filtrate was concentrated and the residue distilled twice to give 2-(5-chloropentyl)-6-acetylfuran, b.p. 100-102 ° C(0.05 mm) in 60% yield.

h) 2-{3,5-Dichloro-4-[5-(6-acetyl-2-furanyl)pentyloxy]phenyl]-4,5-dihydrooxazole [**I**; Y = $(CH_2)_5$, X = O, Z = $CH_3COC$, m = 0, R = H, $R_1$ and $R_2$ = Cl, $R_3$ and $R_4$ = H] can be prepared by reacting 2-(5-chloropentyl)-6-acetylfuran with 2,6-dichloro-4-(4,5-dihydro-2-oxazolyl)phenol in the presence of potassium carbonate and sodium iodide according to the general procedure of Example 1, part (c).

Example 16

5-{5-[2,6-Dichloro-4-(4,5-dihydro-2-oxazolyl)phenoxy]pentyl}-3-isoxazolemethanol [**II**; R = $HOCH_2$, $R_1$ and $R_2$ = Cl, $R_3$ and $R_4$ = H, m = 0], colorless solid, m.p. 65-66 ° C (from hexane-isopropyl acetate).

Intermediates: 2,6-dichloro-4-(4,5-dichloro-2-oxazolyl)phenol and 5-(5-chloropentyl)-3-hydroxymethylisoxazole, prepared from 3-hydroxymethyl-5-methylisoxazole and 1-chloro-4-bromobutane.

Example 17

5-{5-[2,6-Dichloro-4-(4,5-dihydro-2-oxazolyl)phenoxy]pentyl}-3-isoxazolylmethyl methyl butanedioate [**I**; Y = $(CH_2)_5$, X = O, Z = N, m = 0, R = $H_3COOC(CH_2)_2COOCH_2$, $R_1$ and $R_2$ = Cl, $R_3$ and $R_4$ = H].

To a solution of 5.0 g of 5-{5-(2,6-dichloro-4-(4,5-dihydro-2-oxazolyl)phenoxy]pentyl]-3-isoxazolemethanol (Example 16), 1.75 g of monomethyl succinate and a catalytic amount of p-dimethylaminopyridine in 100 ml of chloroform at 0 °C was added 2.6 g of dicyclohexylcarbodiimide. The reaction mixture was stirred at room temperature for 24 hrs, and then filtered and concentrated to give 4.5 g of product which upon recrystallization from isopropyl acetate-hexane gave 4.2 g of 5-{5-[2,6-dichloro-4-(4,5-dihydro-2-oxazolyl)phenoxy]pentyl}-3-isoxazolylmethyl methyl butanedioate, m.p. 39-41 °C.

Example 18

5-{5-[2,6-Dichloro-4-(4,5-dihydro-2-oxazolyl)phenoxy]pentyl}-3-(methoxymethyl)isoxazole [**I**; Y = $(CH_2)_5$, X = O, Z = N, m = 0, R = $CH_3OCH_2$, $R_1$ and $R_2$ = Cl, $R_3$ and $R_4$ = H].

To a solution of 6.8 g of 5-{5-[2,6-dichloro-4-(4,5-dihydro-2-oxazolyl)phenoxy]pentyl}-3-isoxazolemethanol (Example 16) in 100 ml of dry tetrahydrofuran was added 0.45 g of sodium hydride. After hydrogen evolution had ceased, the solution was cooled in an ice-bath and 1.1 ml of methyl iodide was added. The reaction mixture was stirred at room temperature for 48 hrs, then diluted with ether and washed well with water. Evaporation of the solvent and chromatography provided 3.1 g of the product as a viscous yellow oil.

Example 19

a) 5-{5-[2,6-Dichloro-4-(4,5-dihydro-2-oxazolyl)phenoxy]pentyl}-3-isoxazolylmethoxymethoxyethyl acetate [**I**; Y = $(CH_2)_5$, X = O, Z = N, m = 0, R = $CH_3CO(CH_2)_2OCH_2OCH_2$, $R_1$ and $R_2$ = Cl, $R_3$ and $R_4$ = H] was prepared from 4.0 g of 5-{5-[2,6-dichloro-4-(4,5-dihydro-2-oxazolyl)phenoxy]pentyl}-3-isoxazolemethanol (Example 16) and 2.0 g of 2-acetoxyethoxymethyl bromide [$CH_3COCH_2CH_2OCH_2Br$] in the presence of sodium hydride according to the procedure of Example 18, and was obtained in the form of a colorless oil (2.4 g).

b) 5-{5-[2,6-Dichloro-4-(4,5-dihydro-2-oxazolyl)phenoxy]pentyl]-3-isoxazolylmethoxymethoxyethanol [**I**; Y = $(CH_2)_5$, X = O, Z = N, m = 0, R = $HO(CH_2)_2OCH_2OCH_2$, $R_1$ and $R_2$ = Cl, $R_3$ and $R_4$ = H] was prepared by hydrolysis of the acetate of part (a) with lithium hydroxide in methanol, 12 hrs at room temperature and obtained as a pale yellow oil (2.8 g).

It is further contemplated that 5-{5-[2,6-dichloro-4-(4,5-dihydro-2-oxazolyl)phenoxy]pentyl]-3-isoxazolemethanol (Example 16) can be caused to react with thionyl chloride to afford 3-chloromethyl-5-{5-[2,6-dichloro-4-(4,5-dihydro-2-oxazolyl)phenoxy]pentyl}isoxazole [**I**; Y = $(CH_2)_5$, X = O, Z = N, m = 0, R = $ClCH_2$, $R_1$ and $R_2$ = Cl, $R_3$ and $R_4$ = H], and that the latter in turn can be caused to react with ammonia or an amine, e.g. ethylamine, dimethylamine, pyrrolidine, piperidine or morpholine to give the corresponding compounds having a 3-aminomethyl, 3-ethylaminomethyl, 3-dimethylamino, 3-(1-pyrrolidyl)methyl, 3-(1-piperidinyl)methyl or 3-(4-morpholinyl)methyl group.

Example 20

a) 4-(4,5-Dimethyl-2-oxazolyl)-2,6-dimethylphenol N-oxide.

Hydrogen chloride gas was passed through a mixture of 13.45 g 3,5-dimethyl-4-hydroxybenzaldehyde and 9.3 g 2,3-butanedione mono-oxime in 66.7 ml glacial acetic acid for 11 minutes. The reaction mixture was held at room temperature overnight, and the solid product was collected, washed with ether and dried to give 14.86 g 4-(4,5-dimethyl-2-oxazolyl)-2,6-dimethylphenol N-oxide in the form of its hydrochloride salt, m.p. 198-201 °C.

b) 4-(4,5-Dimethyl-2-oxazolyl)-2,6-dimethylphenol [**IX**; $R_1$, $R_2$, $R_3$ and $R_4$ = $CH_3$, m = 0].

To a mixture of 14.86 g 4-(4,5-dimethyl-2-oxazolyl)-2,6-dimethylphenol N-oxide hydrochloride and 220 ml of acetic acid stirred at 100 °C was added portionwise 28 g of zinc dust. The reaction mixture was stirred at 100 °C for 1.5 hr and held at room temperature for three days. The product was isolated by partitioning between ethyl acetate and water. From the ethyl acetate fraction was obtained 11 g of product which was recrystallized from acetonitrile to give 7.31 g of 4-(4,5-dimethyl-2-oxazolyl)-2,6-dimethylphenol, m.p. 203-205 ° C.

c) 5-{5-[4-(4,5-Dimethyl-2-oxazolyl)-2,6-dimethylphenoxy]pentyl}-3-methylisoxazole [**II**; R, $R_1$, $R_2$, $R_3$ and $R_4$ = $CH_3$, m = 0], hydrochloride salt, m.p. 136-140 ° C (colorless granules from ethanol) was prepared from 4-(4,5-dimethyl-2-oxazolyl)-2,6-dimethylphenol and 5-(5-bromopentyl)-3-methylisoxazole.

Example 21

a) 2-(3,5-Dichloro-4-hydroxyphenyl)-4,5-dimethyloxazole N-oxide was prepared from 19.1 g 3,5-dichloro-4-hydroxybenzaldehyde and 10.1 g butanedione mono-oxime in formic acid in the presence of hydrogen chloride gas. There was obtained 12.2 g of product, m.p. 221-223 ° C.

b) 3,5-Dichloro-4-(4,5-dimethyl-2-oxazolyl)phenol [**IX**; $R_1$ and $R_2$ = Cl, $R_3$ and $R_4$ = $CH_3$, m = 0].

Titanium trichloride solution (20%, 100 ml) was added dropwise over a 30 minute period to a stirred mixture of 16.0 g of the N-oxide of part (a) in 1000 ml of tetrahydrofuran. The product was isolated and there was obtained 9.0 g 3,5-dichloro-4-(4,5-dimethyl-2-oxazolyl)phenol, m.p. 228-229 ° C.

c) 5-{5-[2,6-Dichloro-4-(4,5-dimethyl-2-oxazolyl)phenoxy]pentyl}-3-methylisoxazole [**II**; m = 0, R = $CH_3$, $R_1$ and $R_2$ = Cl, $R_3$ and $R_4$ = $CH_3$], m.p. 61-62 ° C (colorless solid from triethylamine), was prepared from 3,5-dichloro-4-(4,5-dimethyl-2-oxazolyl)phenol and 5-(5-bromopentyl)-3-methylisoxazole.

Example 22

5-{4-[2,6-Dichloro-4-(4,5-dimethyl-2-oxazolyl)phenoxy]butyl}-3-methylisoxazole [**I**; Y = $(CH_2)_4$, X = O, Z = N, m = 0, R = $CH_3$, $R_1$ and $R_2$ = Cl, $R_3$ and $R_4$ = $CH_3$], m.p. 64-65 ° C (from ether-hexane) was prepared from 3,5-dichloro-4-(4,5-dimethyl-2-oxazolyl)phenol (Example 21b) and 5-(4-bromobutyl)-3-methylisoxazole.

Example 23

5-{5-[2,6-Dichloro-4-(2-oxazolyl)phenoxy]pentyl}-3-methylisoxazole [**II**; R = $CH_3$, $R_1$ and $R_2$ = Cl, $R_3$ and $R_4$ = H, m = 0], light tan powder, m.p. 39-41 ° C (from ether-hexane).

Example 24

a) Methyl 3-bromo-5-chloro-4-{[5-(3-methyl-5-isoxazolyl)pentyl]oxy}benzoate [**X**; Y = $(CH_2)_5$, Z = N, R = $CH_3$, $R_1$ = Cl, $R_2$ = Br, Alk = $CH_3$] was prepared from 41.3 g methyl 3-bromo-5-chloro-4-hydroxybenzoate (prepared by bromination of 3-chloro-4-hydroxybenzoic acid and esterification), 40.6 g 5-(5-bromopentyl)-3-methylisoxazole, 61.7 g potassium carbonate and 0.2 g sodium iodide in 500 ml of dimethylformamide, 3 hrs at 90-95 ° C, and was obtained in the form of a colorless oil after chromatography on silica.

b) 3-Bromo-5-chloro-4-{[5-(3-methyl-5-isoxazolyl)pentyl]oxy}benzoic acid [**XI**; Y = $(CH_2)_5$, Z = N, R = $CH_3$, $R_1$ = Cl, $R_2$ = Br] was prepared by hydrolysis of 51.3 g of the ester of part (a) with 3.1 g lithium hydroxide in 500 ml methanol and 10 ml water, heated at reflux overnight. The reaction mixture was diluted, acidified and extracted with ethyl acetate. From the latter was obtained 42.8 g product, m.p. 104-105 ° C after recrystallization from ether-hexane.

c) 3-Bromo-5-chloro-N-(2-chloroethyl)-4-{[5-(3-methyl-5-isoxazolyl)pentyl]oxy}benzamide [**III**; X = O, Y = $(CH_2)_5$, Z = N, m = 0, R = $CH_3$, $R_1$ = Cl, $R_2$ = Br, $R_3$ and $R_4$ = H, Hal' = Cl].

The acid of part (b) (29.2 g) was stirred with excess thionyl chloride (about 35 ml) for about 16 hrs. The unreacted thionyl chloride was aspirated off and 250 ml chloroform was added, followed by 21 g 2-chloroethylamine hydrochloride. This mixture was cooled to 0 ° C and 80 ml triethylamine was added dropwise. The reaction mixture was stirred overnight, then diluted with chloroform and washed with dilute hydrochloric acid. From the chloroform solution was obtained 30.9 g product of sufficient purity to be used in the next step. A sample of the compound when purified by chromatography and recrystallized from isopropyl alcohol had the m.p. 84-86 ° C.

d) 5-{5-[2-Bromo-6-chloro-4-(4,5-dihydro-2-oxazolyl)phenoxy]pentyl}-3-methylisoxazole [**II**; R = $CH_3$, $R_1$ = Cl, $R_2$ = Br, $R_3$ and $R_4$ = H, m = 0].

A mixture of 19.0 g of the chloroamide of part (c) and 12.0 g 1,8-diazabicyclo[5.4.0]undec-7-ene in 250 ml methylene dichloride was heated at reflux for two days. The product was isolated, purified through silica gel and recrystallized from ether to give 10.0 g of 5-{5-[2-bromo-6-chloro-4-(4,5-dihydro-2-oxazolyl)phenoxy]pentyl}-3-methylisoxazole, m.p. 41-42 ° C.

The following Examples 25-29 were prepared by procedures analogous to those described in Example 24.

Example 25

a) Methyl 3-chloro-4-{[5-(3-methyl-5-isoxazolyl)pentyl]oxy}-5-nitrobenzoate [**X**; Y = $(CH_2)_5$, Z = N, R = $CH_3$, $R_1$ = Cl, $R_2$ = $NO_2$, Alk = $CH_3$], m.p. 65-67°C (from t-butyl methyl ether).

The intermediate methyl 3-chloro-4-hydroxy-5-nitrobenzoate was a yellow solid, m.p. 118-119°C (from carbon tetrachloride).

b) 3-Chloro-4-{[5-(3-methyl-5-isoxazolyl)pentyl]oxy}-5-nitrobenzoic acid [**XI**; Y = $(CH_2)_5$, Z = N, R = $CH_3$, $R_1$ = Cl, $R_2$ = $NO_2$], m.p. 94-95°C (from hexane-isopropyl acetate).

c) 3-Chloro-N-(2-chloroethyl)-4-{[5-(3-methyl-5-isoxazolyl)pentyl]oxy}-5-nitrobenzamide [**III**; X = O, Y = $(CH_2)_5$, Z = N, m = 0, R = $CH_3$, $R_1$ = Cl, $R_2$ = $NO_2$, $R_3$ and $R_4$ = H, Hal' = Cl], m.p. 73-74°C (from t-butyl methyl ether).

d) 5-{5-[2-Chloro-4-(4,5-dihydro-2-oxazolyl)-6-nitrophenoxy]pentyl}-3-methylisoxazole [II; R = $CH_3$, $R_1$ = Cl, $R_2$ = $NO_2$, $R_3$ and $R_4$ = H, m = 0], viscous yellow oil after chromatography.

Example 26

a) 3-Chloro-5-methyl-4-{[5-(3-methyl-5-isoxazolyl)pentyl]oxy}benzoic acid [**XI**; Y = $(CH_2)_5$, Z = N, R = $CH_3$, $R_1$ = Cl, $R_2$ = $CH_3$], m.p. 98-99°C (from ether-hexane).

The foregoing compound was prepared from 3-chloro-4-hydroxy-5-methylbenzaldehyde, brownish-orange flakes, m.p. 114-116°C (prepared from 2-chloro-6-methylphenol and hexamethylenetetramine in trifluoroacetic acid), by alkylation with 5-(5-bromopentyl)-3-methylisoxazole to produce 3-chloro-5-methyl-4-{[5-(3-methyl-5-isoxazolyl)pentyl]oxy}benzaldehyde, and oxidation of the latter with silver nitrate.

b) 3-Chloro-N-(2-chloroethyl)-5-methyl-4-{[5-(3-methyl-5-isoxazolyl)pentyl]oxy}benzamide [**III**; X = O, Y = $(CH_2)_5$, Z = N, m = 0, R = $CH_3$, $R_1$ = Cl, $R_2$ = $CH_3$, $R_3$ and $R_4$ = H, Hal' = Cl], m.p. 84-85°C (from ether).

c) 5-{5-[2-Chloro-4-(4,5-dihydro-2-oxazolyl)-6-methylphenoxy]pentyl}-3-methylisoxazole [**II**; R = $CH_3$, $R_1$ = Cl, $R_2$ = $CH_3$, $R_3$ and $R_4$ = H, m = 0], colorless liquid.

Example 27

a) 3-Chloro-4-{[5-(3-methyl-5-isoxazolyl)pentyl]oxy}-5-trifluoromethylbenzoic acid [**XI**; Y = $(CH_2)_5$, Z = N, R = $CH_3$, $R_1$ = Cl, $R_2$ = $CF_3$].

The foregoing compound was prepared by chlorination of 3-trifluoromethyl-4-hydroxybenzonitrile, alkylation of the latter with 5-(5-iodopentyl)-3-methylisoxazole to produce 3-chloro-4-{[5-(3-methyl-5-isoxazolyl)pentyl]oxy}-5-trifluoromethylbenzonitrile, followed by hydrolysis of the nitrile group to a carboxy group.

b) 3-Chloro-N-(2-chloroethyl)-4-{[5-(3-methyl-5-isoxazolyl)pentyl}-5-trifluoromethylbenzamide [**III**; X = O, Y = $(CH_2)_5$, Z = N, m = 0, R = $CH_3$, $R_1$ = Cl, $R_2$ = $CF_3$, $R_3$ and $R_4$ = H, Hal' = Cl].

c) 5-{5-[2-Chloro-4-(4,5-dihydro-2-oxazolyl)-6-(trifluoromethyl)phenoxy]pentyl}-3-methylisoxazole [**II**; R = $CH_3$, $R_1$ = Cl, $R_2$ = $CF_3$, $R_3$ and $R_4$ = H, m = 0], pale yellow liquid by chromatography.

Example 28

a) 3,5-Dichloro-N-(3-chloropropyl)-4-{[5-(3-methyl-5-isoxazolyl)pentyl]oxy}benzamide [**III**; X = O, Y = $(CH_2)_5$, Z = N, m = l, R = $CH_3$, $R_1$ and $R_2$ = Cl, $R_3$ and $R_4$ = H, Hal' = Cl], colorless needles, m.p. 72-73°C.

b) 5,6-Dihydro-2-{4-[5-(3-methyl-5-isoxazolyl)pentyloxy]phenyl}-4H-1,3-oxazine [**II**; R = $CH_3$, $R_1$ and $R_2$ = Cl, $R_3$ and $R_4$ = H, m = l], colorless oil.

Example 29

a) Methyl 3,5-dichloro-4-{[5-(3-methyl-5-isoxazolyl)pentyl]oxy}benzoate [**X**; Y = $(CH_2)_5$, Z = N, R = $CH_3$, $R_1$ and $R_2$ = Cl, Alk = $CH_3$], pale yellow oil by chromatography.

b) 3,5-Dichloro-4-{[5-(3-methyl-5-isoxazolyl)pentyl]oxy}benzoic acid [**XI**; Y = $(CH_2)_5$, Z = N, R = $CH_3$, $R_1$ and $R_2$ = Cl], m.p. 77-79°C.

c) 3,5-Dichloro-N-(2-chloroethyl)-4-{[5-(3-methyl-5-isoxazolyl)pentyl]oxy}benzamide [**III**; X = O, Y = $(CH_2)_5$, Z = N, m = 0, R = $CH_3$, $R_1$ and $R_2$ = Cl, $R_3$ and $R_4$ = H, Hal' = Cl], m.p. 88-90°C.

d) 5-{5-[2,6-Dichloro-4-(4,5-dihydro-2-oxazolyl)phenoxy]pentyl}-3-methylisoxazole [**II**; R = $CH_3$, $R_1$ and

$R_2$ = Cl, $R_3$ and $R_4$ = H, m = 0].

To a solution of 1.0 g 3,5-dichloro-N-(2-chloroethyl)-4-{[5-(3-methyl-5-isoxazolyl)pentyl]-oxy}benzamide in 75 ml methylene dichloride was added 0.92 g 1,8-diazabicyclo[5.4.0]undec-7-ene, and the mixture was heated at reflux for about 16 hrs. The reaction mixture was concentrated in vacuo and the residue partitioned between 150 ml ethyl acetate and 50 ml water. The organic layer was separated, washed with water and saturated sodium chloride solution, dried over anhydrous magnesium sulfate and concentrated in vacuo. The residue was chromatographed on silica gel and eluted with 1:1 ethyl acetate:hexane. The eluted material was isolated, dissolved in ether and crystallized upon seeding to give 0.60 g (66%) of 5-{5-[2,6-dichloro-4-(4,5-dihydro-2-oxazolyl)phenoxy]pentyl}-3-methylisoxazole, light yellow solid, m.p. 39-40 °C, identical with the compound of Example 1(c), as confirmed by thin layer chromatography.

Example 30

a) Ethyl 3,5-dichloro-4-[6-(3-methyl-5-isoxazolyl)hexyl]benzoate [**XIV**; Y = $(CH_2)_5$, Z = N, R = $CH_3$, $R_1$ and $R_2$ = Cl, Alk = $C_2H_5$].

To a solution of 1.2 ml hexamethylphosphoramide and 6.7 ml 1.75 M lithium diisopropylamide in 9 ml tetrahydrofuran at 0 °C under nitrogen was slowly added a solution of 1.00 g 3,5-dichloro-4-methylbenzoic acid in 3 ml tetrahydrofuran. After 1 hr the solution was cooled to -78 °C and 1.00 g 5-(5-bromopentyl)-3-methylisoxazole was added. The reaction mixture was stirred at room temperature for 19 hrs and worked up by acid-base extraction to give 1.3 g crude acid product. The latter was treated with 1 ml ethyl iodide and 4.9 g potassium carbonate in 10 ml dimethylformamide, stirred 12 hrs at room temperature. Extraction with ether and washing with water provided 1 g of ethyl 3,5-dichloro-4-[6-(3-methyl-5-isoxazolyl)hexyl]benzoate suitable for subsequent reactions.

b) 5-{6-[2,6-Dichloro-4-(4,5-dihydro-2-oxazolyl)phenyl]hexyl}-3-methylisoxazole [**I**; X = $CH_2$, Y = $(CH_2)_5$, Z = N, R = $CH_3$, $R_1$ and $R_2$ = Cl, $R_3$ and $R_4$ = H].

A solution of 2.6 g ethyl 3,5-dichloro-4-[6-(3-methyl-5-isoxazolyl)hexyl]benzoate and 0.21 g lithium hydroxide in 30 ml methanol and 10 ml water was heated at reflux for 12 hrs. Work-up provided 2.2 g of the corresponding benzoic acid which was stirred with 2 ml thionyl chloride in chloroform for 14 hrs. The mixture was concentrated and added to a solution of 1.00 g ethanolamine in 50 ml of methylene dichloride. Flash filtration provided the 2-hydroxyethylamide (1.7 g) as a yellow solid. The latter was converted to its methanesulfonate ester with 0.33 ml methanesulfonyl chloride and 0.7 ml triethylamine in methylene dichloride, and said mesylate was dissolved in 100 ml acetonitrile containing 1.0 g 1,8-diazabicyclo[5.4.0]undec-7-ene and heated at reflux for 4 hrs. Concentration and flash filtration provided 1.0 g 5-{6-[2,6-dichloro-4-(4,5-dihydro-2-oxazolyl)phenyl]hexyl}-3-methylisoxazole, colorless solid, m.p. 65-66 °C when recrystallized from isopropyl acetate-hexane.

Alternatively, the 2-hydroxyethylamide intermediate was treated with thionyl chloride to form N-(2-chloroethyl)-3,5-dichloro-4-[6-(3-methyl-5-isoxazolyl)hexyl]benzamide [**III**; X = $CH_2$, Y = $(CH_2)_5$, Z = N, m = 0, R = $CH_3$, $R_1$ and $R_2$ = Cl, $R_3$ and $R_4$ = H, Hal' = Cl], and the latter then cyclized to the final product.

Biological evaluation of compounds of Formulas **I** and **III** has shown that they possess antiviral activity. They are useful in inhibiting virus replication in vitro and are primarily active against picornaviruses, especially numerous strains of rhinoviruses. The in vitro testing of the compounds of the invention against picornaviruses showed that viral replication was inhibited at minimum inhihitory concentrations (MIC) ranging from about 0.003 to about 3 micrograms per milliliter.

The MIC values were determined by a standard plaque reduction assay as follows: HeLa (Ohio) cells in monolayers were infected at a concentration of virus to give approximately 80 plaques per monolayer in the virus control (no drug present). The compound to be tested was serially diluted and included in the agar-medium overlay and in some cases, during the adsorption period as well. The MIC was determined to be that concentration of compound which reduced the number of plaques by 50% with respect to the untreated virus control.

In the standard test procedure, the compounds were tested against a panel of fifteen human rhinovirus (HRV) serotypes, namely HRV-2, -1A, -1B, -6, -14, -21, -22, -15, -25, -30 -50, -67, -89, -86 and -41. The MIC value for each rhinovirus serotype was determined, and the efficacy of each compound was determined in terms of a $MIC_{80}$ value, which is the concentration of the compound required to inhibit 80% of the tested serotypes.

In the in vivo studies, white Swiss mice averaging 20 g in weight were infected with 2 $LD_{50}$'s of poliovirus, type 2, MEF strain, by injecting 0.03 ml of the virus (540 pfu) into the left cerebral

hemisphere. The mice were medicated intragastrically with the test compound as a suspension in gum tragacanth one hour prior to infection, 6 hrs post-infection and then b.i.d. for a total of 10 days. Appropriate placebo-medicated mice were included in the test, and all mice were checked twice daily for deaths. The test was terminated at 14 days post-infection.

The blood plasma concentrations of the compounds of the invention were measured in beagle dogs by the following procedure: Animals ranging in weight from 8.5 to 11.5 kg were fasted overnight prior to oral administration of 25 mg/kg of the test compound dissolved in corn oil (125 mg/ml) and contained in gelatin capsules. Blood samples were collected at selected time intervals up to six hours post-medication and analyzed by standard chromatographic procedures to determine the mean maximum concentration of the test compound in micrograms per milliliter [Mean $C_{max}$, µg/ml].

The following Table gives the testing results with the compounds of the invention.

A. Compounds of Formula I

| Example No. | MIC (Polio 2) | $MIC_{80}$ (Rhinovirus) | Plasma Concentration (Dog) Mean $C_{max}$, µg/ml |
|---|---|---|---|
| 1c | 0.59 | 0.126 | 1.57 ± 0.35 |
| 2 | 3.1 | 0.43 | 4.26 ± 0.47 |
| 3 | 2 | 0.59 | |
| 4 | 0.58 | 0.41 | 1.53 ± 0.15 |
| 5 | 4.6 | 0.37 | 1.53 ± 0.14 |
| 6 | 2.6 | 0.34 | |
| 7 | 2.9 | 0.89 | 2.11 ± 0.61 |
| 8 | IA | 0.64 | |
| 9 | 2.1 | 0.3 | |
| 10 | IA | 0.74 | |
| 11 | 0.4 | 0.06[a] | |
| 12 | 0.48 | 0.12[a] | |
| 13 | 3.1 | 0.04[a] | |
| 14 | 12.5 | 2.0[b] | |
| 16 | 3.1 | 0.5 | |
| 17 | 8.5 | 0.14[a] | |
| 18 | 0.39 | 0.02[a] | |
| 19b | IA | 1.8[b] | |
| 20c | IA | 0.84 | |
| 21c | IA | 0.53 | 2.13 ± 0.48 |
| 22 | IA | 0.67 | |

[contd.]

A. Compounds of Formula I [contd.]

| Example No. | MIC (Polio 2) | $MIC_{80}$ (Rhinovirus) | Plasma Concentration (Dog) Mean $C_{max}$, µg/ml |
|---|---|---|---|
| 23 | 0.82 | 0.45(c) | |
| 24d | 0.97 | 0.23 | |
| 25d | 0.4 | 0.03(a) | |
| 26 | 0.8 | 0.33 | |
| 27c | IA | 0.56 | |
| 28b | IA | NT | |
| 30 | 1.4 | 0.73 | |

| B. Compounds of Formula III | | |
|---|---|---|
| Example No. | MIC (Polio 2) | $MIC_{80}$ (Rhinovirus) |
| 24c | 2.1 | 0.6 |
| 25c | 0.86 | 0.08(a) |
| 26b | 1.13 | 0.63 |
| 28a | IA | NT |
| 29c | 0.48 | 0.28 |

IA = Inactive
(a) = Against HRV-2 only
(b) = Against 5 HRV serotypes
(c) = Against 6 HRV serotypes
NT = Not yet tested

The compound of Example 1(c), 5-{5-[2,6-dichloro-4-(4,5-dihydro-2-oxazolyl)phenoxy]pentyl}-3-methylisoxazole was tested in mice against poliovirus-2 with the following results:

| Survivors - Day 14 Post Infection | |
|---|---|
| 50 mg/kg/day | 2/20 |
| 100 mg/kg/day | 5/20 |
| 200 mg/kg/day | 6/20 |
| placebo | 1/20 |

In several instances it has been found that the compounds of Formulas **I** and **II** have improved anti-rhinovirus $MIC_{80}$ values and/or plasma concentration $C_{max}$ values as compared with the corresponding compounds having only one substituent or no substituent on the phenyl ring adjacent to the X linkage.

This improvement is particularly apparent from a comparison of the test results with the following three compounds:

A. 5-{5-[2,6-Dichloro-4-(4,5-dihydro-2-oxazolyl)phenoxy]pentyl}-3-methylisoyxazole (Example 1c)

B. 5-{5-[2-Chloro-4-(4,5-dihydro-2-oxazolyl)phenoxy]pentyl}-3-methylisoxazole (European Pat. Appln. 137,242, Example 15b)

C. 5-{5-[4-(4,5-Dihydro-2-oxozolyl)phenoxy]pentyl}-3-methylisoxazole (European Pat. Appln. 137,242, Example 2c).

| Compound | $MIC_{80}$ | $C_{max}$ |
|---|---|---|
| A | 0.23[(a)] | 1.57 ± 0.35 |
| B | 0.57[(a)] | 0.17 ± 0.10 |
| C | 2.2[(b)] | |

(a) against 53 rhinovirus serotypes
(b) against 15 rhinovirus serotypes

In a further instance it was found the compound of Example 2, 5-{5-[4-(4,5-dihydro-2-oxazolyl)-2,6-dimethylphenoxy]pentyl}-3-methylisoxazole, had a plasma concentration $C_{max}$ value of 4.26 ± 0.47 whereas the corresponding mono-methyl analog, 5-[5-[4-(4,5-dihydro-2-oxazolyl)-2-methylphenoxy]pentyl}-3-methylisoxazole had a $C_{max}$ value of only about 0.50.

Additional compounds which have unusually high in vitro antirhinovirus activity are those of Examples 9, 24(d) and 26 (see above Table).

The antiviral compositions are formulated for use by preparing a dilute solution or suspension in a pharmaceutically acceptable aqueous, organic or aqueous-organic medium for parenteral administration by intravenous or intramuscular injection, or for intranasal or ophthalmic application; or are prepared in tablet, capsule, or aqueous suspension form with conventional excipients for oral administration.

## Claims

**Claims for the following Contracting States : BE, CH, DE, FR, GB, IT, LI, NL, SE**

**1.** A compound of the formula

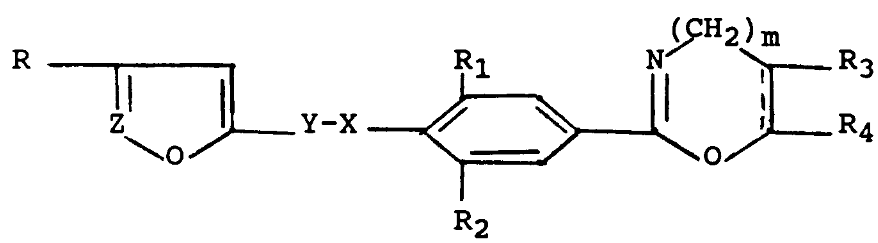

wherein:

R is hydrogen or alkyl of 1 to 3 carbon atoms optionally substituted by hydroxy or alkoxy of 1 to 4 carbon atoms,

X is O or $CH_2$;

Y is an alkylene bridge of 3-9 carbon atoms, or an olefinic linkage of 5 carbon atoms with one unsaturated bond;

Z is N or $R_5C$, where $R_5$ is hydrogen or an alkanoyl of 1-4 carbon atoms with the proviso that when Z is N, R is other than hydrogen;

m is 0 or 1;

$R_1$ and $R_2$ are each halogen, methyl, nitro, or trifluoromethyl; and

$R_3$ and $R_4$ are each hydrogen or lower-alkyl of 1-3 carbon atoms; or

pharmaceutically acceptable acid-addition salts thereof.

**2.** A compound according to claim 1, of the formula

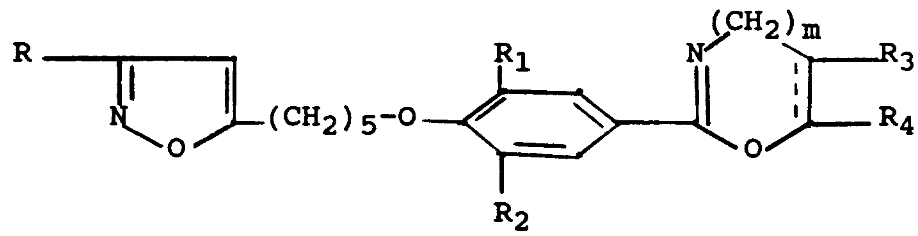

wherein:
m is 0 or 1;
R is methyl or hydroxymethyl;
$R_1$ and $R_2$ are each halogen, methyl, nitro, or trifluoromethyl; and
$R_3$ and $R_4$ are each hydrogen or lower-alkyl of 1-3 carbon atoms; or
pharmaceutically acceptable acid-addition salts thereof.

**3.** A compound according to claim 2, 5-{5-[2,6-dichloro-4-(4,5-dihydro-2-oxazolyl)phenoxy]pentyl)isoxazole or a pharmaceutically acceptable acid-addition salt thereof.

**4.** A compound of the formula

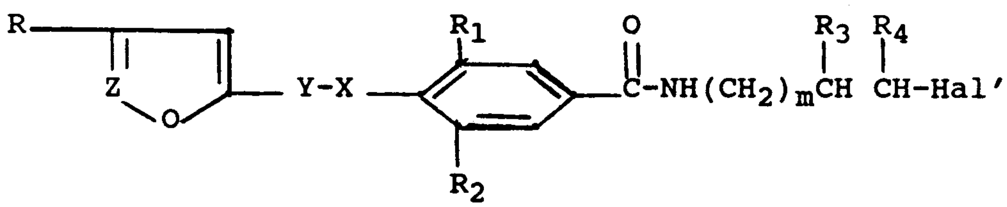

wherein:
Hal' is chlorine or bromine;
X is O or $CH_2$;
Y is an alkylene bridge of 3-9 carbon atoms;
Z is N or $R_5C$, where $R_5$ is hydrogen or lower-alkanoyl of 1-4 carbon atoms;
m is 0 or 1;
$R_1$ and $R_2$ are halogen, methyl, nitro, lower-alkoxycarbonyl of 1-4 carbon atoms or trifluoromethyl; and
R, $R_3$ and $R_4$ are hydrogen or lower-alkyl of 1-3 carbon atoms with the proviso that when Z is N, R is lower-alkyl of 1-4 carbon atoms.

**5.** A composition for combating viruses which comprises an antivirally effective amount of a compound according to any one of claims 1-4, in admixture with a suitable carrier or diluent.

**6.** A process for preparing a compound of the formula

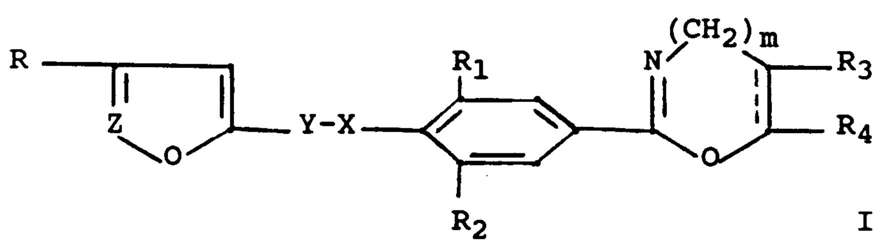

I

wherein:
X is O or $CH_2$;

Y is an alkylene bridge of 3-9 carbon atoms, or an olefinic linkage of 5 carbon atoms with one unsaturated bond; optionally interrupted by an olefinic linkage;

Z is N or $R_5C$, where $R_5$ is hydrogen or lower alkanoyl of 1-4 carbon atoms with the proviso that when Z is N, R is alkyl of 1-3 carbon atoms;

m is 0 or 1;

R is hydrogen or alkyl of 1 to 3 carbon atoms optionally substituted by hydroxy, or lower alkoxy of 1-4 carbon atoms;

$R_1$ and $R_2$ are halogen, methyl, nitro, or trifluoromethyl; and

$R_3$ and $R_4$ are hydrogen or lower-alkyl of 1-3 carbon atoms;

which comprises

(a) to prepare a compound without a double bond in the 4,5-position of the oxazole or 5,6 oxazine ring and wherein R is hydrogen or lower-alkyl of 1-3 carbon atoms cyclizing a compound of the formula

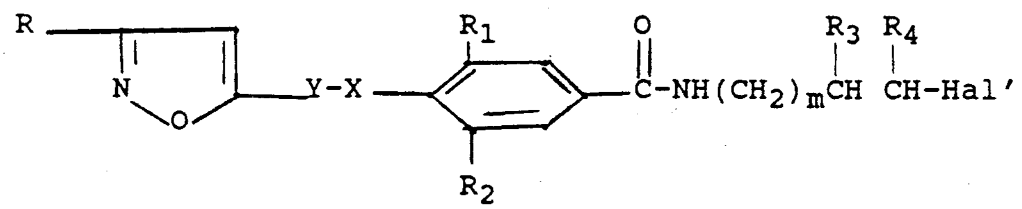

wherein Hal' is chlorine or bromine, by heating the latter in an inert solvent in the presence of an acid-acceptor, excluding compounds wherein Y is an alkylene bridge interrupted by an olefinic linkage; or

(b) to prepare a compound wherein X is O, reacting a compound of the formula

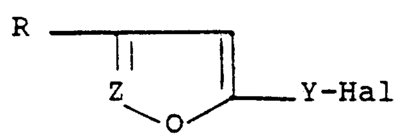

wherein Hal is chlorine, bromine or iodine, with an alkali metal salt of a compound of the formula

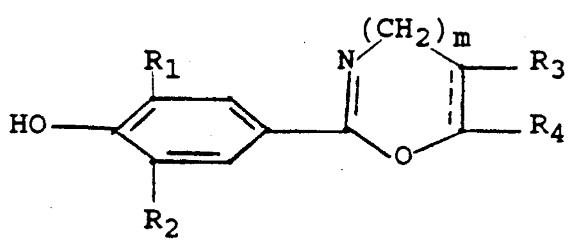

,

if desired, etherifying a compound of Formula I wherein R is hydroxyalkyl by reacting with a lower-alkyl halide in the presence of a strong base to provide a compound wherein R is lower alkoxy lower-alkyl, lower measuring $C_1$-$C_4$

and, if desired, converting a basic compound obtained to an acid-addition salt thereof.

**Claims for the following Contracting State : AT**

**1.** A process for preparing a compound of the formula

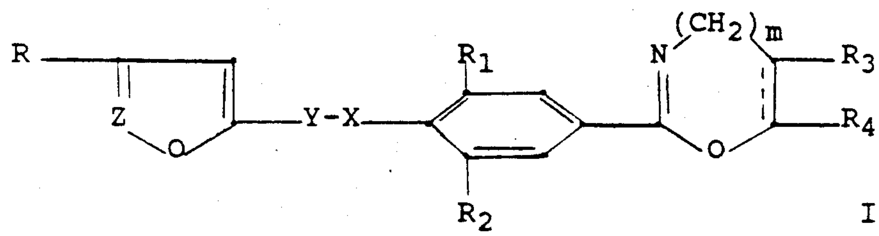

I

wherein:

X is O or $CH_2$;

Y is an alkylene bridge of 3-9 carbon atoms, or an olefinic linkage of 5 carbon atoms with one unsaturated bound;

Z is N or $R_5C$, where $R_5$ is hydrogen or lower alkanoyl of 1-4 carbon atoms, with the proviso that when Z is N, R is alkyl of 1-3 carbon atoms;

m is 0 or 1;

R is hydrogen or alkyl of 1 to 3 carbon atoms optionally substituted by hydroxy, or lower alkoxy of 1-4 carbon atoms;

$R_1$ and $R_2$ are halogen, methyl, nitro, or trifluoromethyl; and

$R_3$ and $R_4$ are hydrogen or lower-alkyl of 1-3 carbon atoms; characterized in that it comprises

(a) to prepare a compound without a double bond in the 4,5-position of the oxazole or the 5,6 oxazine ring and wherein R is hydrogen or lower-alkyl of 1-3 carbon atoms cyclizing a compound of the formula

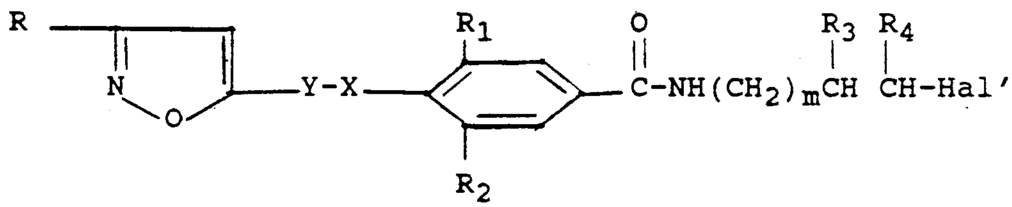

wherein Hal' is chlorine or bromine, by heating the latter in an inert solvent in the presence of an acid-acceptor, excluding compounds wherein Y is an alkylene bridge interrupted by an olefinic linkage; or

(b) to prepare a compound wherein X is O, reacting a compound of the formula

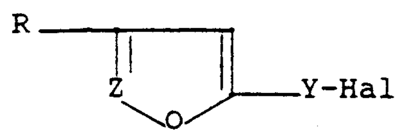

wherein Hal is chlorine, bromine or iodine, with an alkali metal salt of a compound of the formula

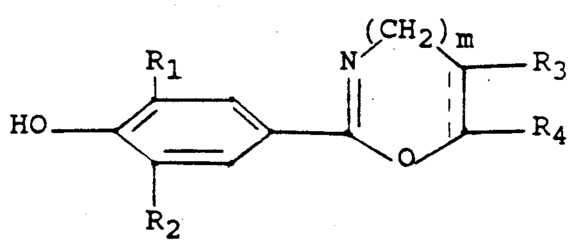

,

if desired, etherifying a compound of Formula I wherein R is hydroxyalkyl by reacting with a lower-alkyl halide of 1-4 carbon atoms in the presence of a strong base to provide a compound wherein R is lower alkoxyalkyl, respectively;

and, if desired, converting a basic compound obtained to an acid-addition salt thereof.

**2.** A process according to claim 1, characterized by preparing a compound of the formula

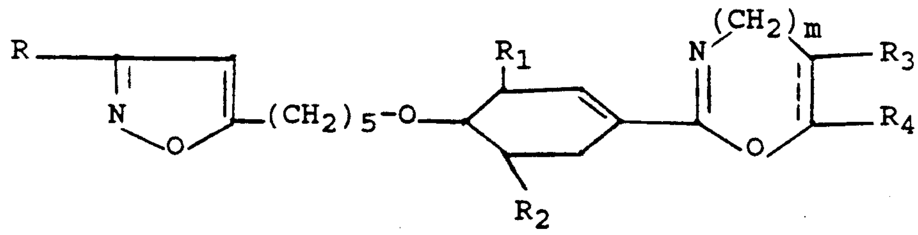

wherein:
m is 0 or 1;
R is methyl or hydroxymethyl;
$R_1$ and $R_2$ are each halogen, methyl, nitro, or trifluoromethyl; and
$R_3$ and $R_4$ are each hydrogen or lower-alkyl of 1-3 carbon atoms; or
pharmaceutically acceptable acid-addition salts thereof.

**3.** A process according to claim 2, characterized by preparing 5-{5-[2,6-dichloro-4-(4,5-dihydro-2-oxazolyl)phenoxy]pentyl)isoxazole or a pharmaceutically acceptable acid-addition salt thereof.

**Patentansprüche**
**Patentansprüche für folgende Vertragsstaaten : BE, CH, DE, FR, GB, IT, LI, NL, SE**

**1.** Verbindung der Formel

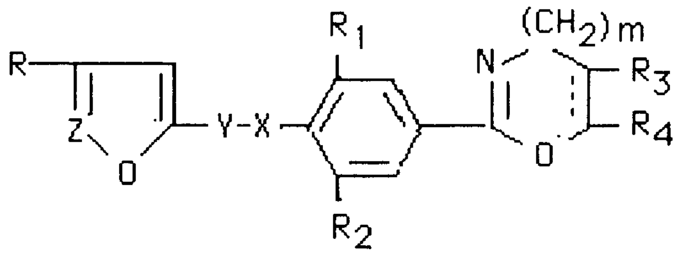

worin sind:

| | |
|---|---|
| R | Wasserstoff oder Alkyl mit 1 bis 3 Kohlenstoffatomen, die wahlweise durch Hydroxy oder Alkoxy mit 1 bis 4 Kohlenstoffatomen substituiert sind; |
| X | O oder $CH_2$; |
| Y | eine Alkylenbrücke mit 3 bis 9 Kohlenstoffatomen oder eine Olefinverkettung Voll 5 Kohlenstoffatomen mit einer ungesättigten Bindung; |
| Z | N oder $R_5C$, worin $R_5$ Wasserstoff ist oder ein Alkanoyl mit 1 bis 4 Kohlenstoffatomen unter der Voraussetzung, daß R nicht Wasserstoff ist, wenn Z N ist; |
| m | O oder 1; |
| $R_1$ und $R_2$ | jeweils Halogen, Methyl, Nitro oder Trifluormethyl und |
| $R_3$ und $R_4$ | jeweilsWasserstoff oder niederes Alkyl mit 1 bis 3 Kohlenstoffatomen oder deren pharmazeutisch zulässige Säureanlagerungssalze. |

**2.** Verbindung nach Anspruch 1 der Formel

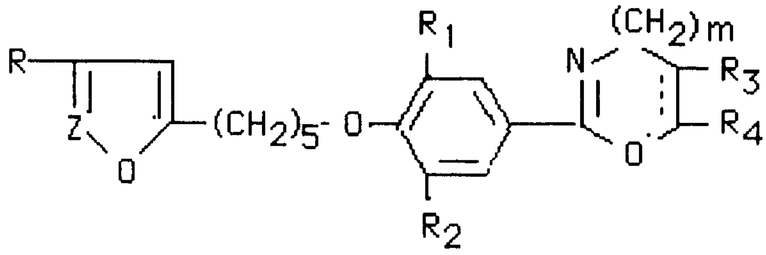

worin sind:

m 0 oder 1;

R Methyl oder Hydroxymethyl;

$R_1$ und $R_2$ jeweils Halogen, Methyl, Nitro oder Trifluormethyl und

$R_3$ und $R_4$ jeweils Wasserstoff oder niederes Alkyl mit 1 bis 3 Kohlenstoffatomen oder deren pharmazeutisch zulässige Säureanlagerungssalze.

**3.** Verbindung nach Anspruch 2, 5-{5[2,6-Dichlor-4(4,5-dihydro-2-oxazolyl)phenoxy]pentyl}isoxazol oder dessen pharmazeutisch zulässiges Säureanlagerungssalz.

**4.** Verbindung der Formel

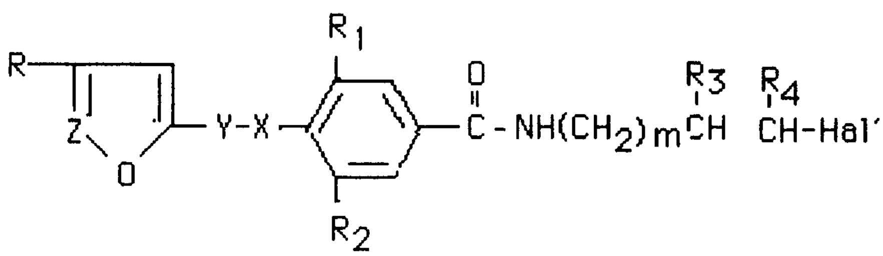

worin sind:

Hal' Chlor oder Brom;

X O oder $CH_2$;

Y Y eine Alkylenbrücke mit 3 bis 9 Kohlenstoffatomen

Z N oder $R_5C$, worin $R_5$ Wasserstoff oder Alkanoyl mit 1 bis 4 Kohlenstoffatomen ist

m 0 oder 1;

$R_1$ und $R_2$ jeweils Halogen, Methyl, Nitro, niederes Alkoxycarbonyl mit 1 bis 4 Kohlenstoffatomen oder Trifluormethyl und

R, $R_3$ und $R_4$ Wasserstoff oder niederes Alkyl mit 1 bis 3 Kohlenstoffatomen unter der Voraussetzung, daß R niederes Alkyl mit 1 bis 4 Kohlenstoffatomen ist, wenn Z N ist.

**5.** Zusammensetzung zur Bekämpfung von Viren umfassend eine Menge einer Verbindung mit antiviraler Wirkung nach einem der Ansprüche 1 bis 4 in Zumischung mit einem geeigneten Träger oder Verdünnungsmittel.

**6.** Verfahren zur Herstellung einer Verbindung der Formel

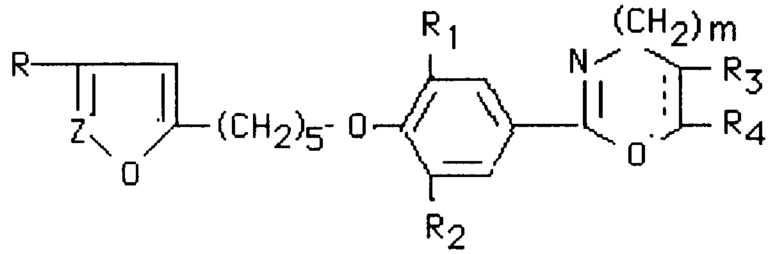

I

worin sind:

| | |
|---|---|
| X | O oder $CH_2$; |
| Y | eine Alkylenbrücke mit 3 bis 9 Kohlenstoffatomen oder eine Olefinverkettung von 5 Kohlenstoffatomen mit einer ungesättigten Bindung; |
| Z | N oder $R_5C$, worin $R_5$ Wasserstoff ist oder niederes Alkanoyl mit 1 bis 4 Kohlenstoffatomen ist, unter der Voraussetzung, daß R Alkyl mit 1 bis 3 Kohlenstoffatomen ist, wenn Z N ist; |
| m | O oder 1; |
| R | Wasserstoff oder Alkyl mit 1 bis 3 Kohlenstoffatomen, das wahlweise durch Hydroxy oder niederes Alkoxy mit 1 bis 4 Kohlenstoffatomen substituiert sind; |
| $R_1$ und $R_2$ | Halogen, Methyl, Nitro oder Trifluormethyl und |
| $R_4$ und $R_4$ | Wasserstoff oder niederes Alkyl mit 1 |

bis 3 Kohlenstoffatomen, welches umfaßt:

(a) Darstellen einer Verbindung ohne eine Doppelbindung in der 4,5-Position des Oxazols oder 5,6-Oxazin-Ringes und worin R Wasserstoff oder niederes Alkyl mit 1 bis 3 Kohlenstoffatomen ist und unter Ringschluß eine Verbindung der Formel

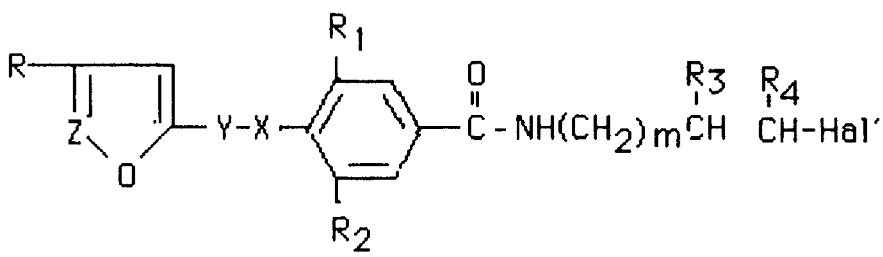

gebildet wird, worin Hal' Chlor oder Brom ist, durch Erhitzen der Letzteren in einem inerten Lösungsmittel in Gegenwart eines Säureakzeptors, ausgenommen Verbindungen, in welchen Y eine Alkylenbrücke ist, die durch eine Olefinverkettung unterbrochen ist; oder

(b) Darstellen einer Verbindung, worin X O ist, indem eine Verbindung der Formel

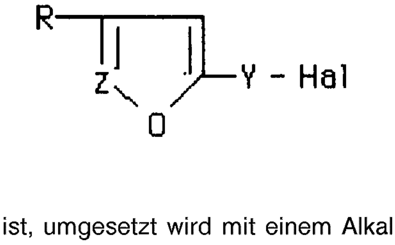

worin Hal Chlor, Brom oder Jod ist, umgesetzt wird mit einem Alkalimetallsalz einer Verbindung der Formel

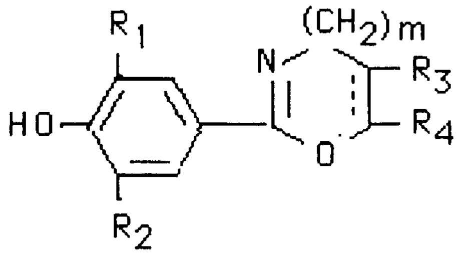

und nach Wunsch Verethern einer Verbindung der Formel I, worin R Hydroxyalkyl ist, durch Umsetzen mit einem niederen Alkylhalogenid in Gegenwart einer starken Base, um eine Verbindung zu schaffen, in welcher R ein niederes Alkoxy - niederes Alkyl ist und "niederes" $C_1$ bis $C_4$ bedeutet;

und nach Wunsch Umwandeln einer erhaltenen basischen Verbindung in ihr Säureanlagerungssalz.

**Patentansprüche für folgenden Vertragsstaat : AT**

**1.** Verfahren zur Herstellung einer Verbindung der Formel

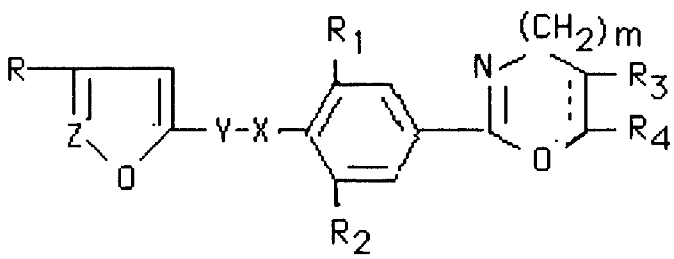

I

worin sind:

| | |
|---|---|
| X | O oder $CH_2$; |
| Y | eine Alkylenbrücke mit 3 bis 9 Kohlenstoffatomen oder eine Olefinverkettung von 5 Kohlenstoffatomen mit einer ungesättigten Bindung; |
| Z | N oder $R_5C$, worin $R_5$ Wasserstoff ist oder niederes Alkanoyl mit 1 bis 4 Kohlenstoffatomen ist, unter der Voraussetzung, daß R Alkyl mit 1 bis 3 Kohlenstoffatomen ist, wenn Z N ist; |
| m | O oder 1; |
| R | Wasserstoff oder Alkyl mit 1 bis 3 Kohlenstoffatomen, das wahlweise durch Hydroxy oder niederes Alkoxy mit 1 bis 4 Kohlenstoffatomen substituiert sind; |
| $R_1$ und $R_2$ | Halogen, Methyl, Nitro oder Trifluormethyl und |
| $R_3$ und $R_4$ | Wasserstoff oder niederes Alkyl mit 1 bis 3 Kohlenstoffatomen, |

dadurch gekennzeichnet, daß es umfaßt:

(a) Darstellen einer Verbindung ohne eine Doppelbindung in der 4,5-Position des Oxazols oder 5,6-Oxazin-Ringes und worin R Wasserstoff oder niederes Alkyl mit 1 bis 3 Kohlenstoffatomen ist und unter Ringschluß eine Verbindung der Formel

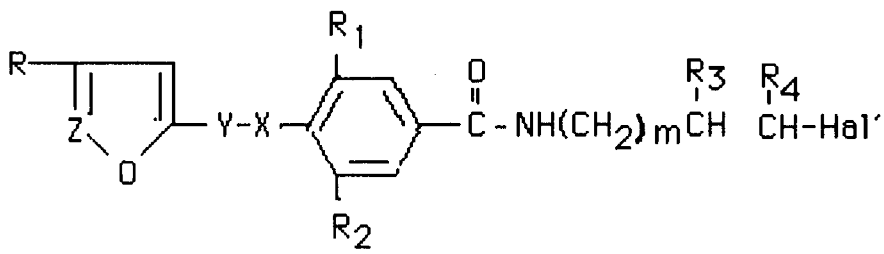

gebildet wird, worin Hal' Chlor oder Brom ist, durch Erhitzen der Letzteren in einem inerten Lösungsmittel in Gegenwart eines Säureakzeptors, ausgenommen Verbindungen, in welchen Y eine Alkylenbrücke ist, die durch eine Olefinverkettung unterbrochen ist; oder

(b) Darstellen einer Verbindung, worin X O ist, indem eine Verbindung der Formel

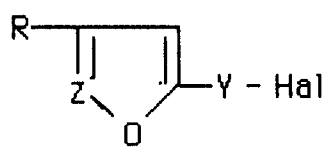

worin Hal Chlor, Brom oder Jod ist, umgesetzt wird mit einem Alkalimetallsalz einer Verbindung der Formel

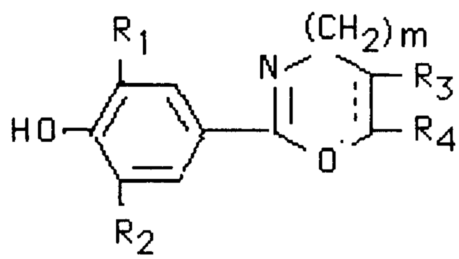

,

und nach Wunsch Verethern einer Verbindung der Formel I, worin R Hydroxyalkyl ist, durch Umsetzen mit einem niederen Alkylhalogenid in Gegenwart einer starken Base, um eine Verbindung zu schaffen, in welcher R jeweils ein niederes Alkoxyalkyl ist

und nach Wunsch Umwandeln einer erhaltenen basischen Verbindung in ihr Säureanlagerungssalz.

**2.** Verfahren nach Anspruch 1, dadurch gekennzeichnet, daß eine Verbindung der Formel

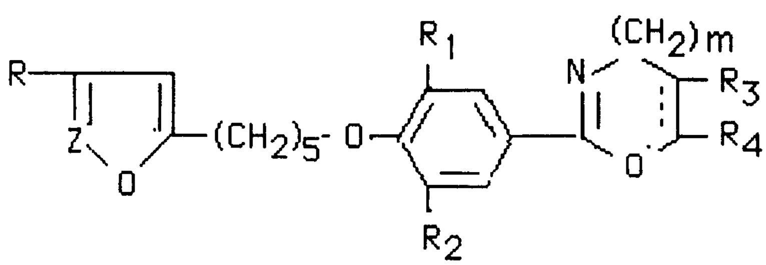

hergestellt wird, worin sind:

| | |
|---|---|
| m | 0 oder 1; |
| R | Methyl oder Hydroxymethyl; |
| $R_1$ und $R_2$ | jeweils Halogen, Methyl, Nitro oder Trifluormethyl und |

$R_3$ und $R_4$ jeweils Wasserstoff oder niederes Alkyl mit 1 bis 3 Kohlenstoffatomen oder deren pharmazeutisch zulässige Säureanlagerungssalze.

3. Verfahren nach Anspruch 1, dadurch gekennzeichnet, daß 5-{5[2,6-Dichlor-4(4,5-dihydro-2-oxazolyl)-phenoxy]pentyl}isoxazol oder dessen pharmazeutisch zulässiges Säureanlagerungssalz hergestellt wird.

**Revendications**

**Revendications pour les Etats contractants suivants : BE, CH, DE, FR, GB, IT, LI, NL, SE**

1. Composé de formule :

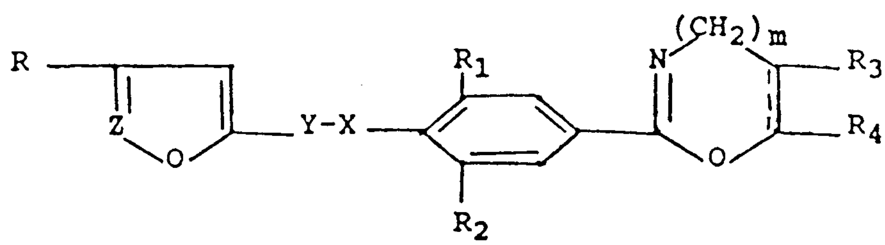

dans laquelle :

R est l'hydrogène ou un alkyle de 1 à 3 atomes de carbone éventuellement substitué par hydroxy ou un alcoxy de 1 à 4 atomes de carbone,

X est 0 ou $CH_2$,

Y est un pont alkylène de 3 à 9 atomes de carbone ou un chaînon oléfinique de 5 atomes de carbone comportant une liaison insaturée,

Z est N ou $R_5C$, où $R_5$ est l'hydrogène ou un alcanoyle de 1 à 4 atomes de carbone, à condition que lorsque Z est N, R ne soit pas l'hydrogène,

m est 0 ou 1,

$R_1$ et $R_2$ sont chacun un halogène, méthyle, nitro ou trifluorométhyle, et

$R_3$ et $R_4$ sont chacun l'hydrogène ou un alkyle inférieur de 1 à 3 atomes de carbone, ou ses sels d'addition d'acide pharmaceutiquement acceptables.

2. Composé selon la revendication 1, de formule

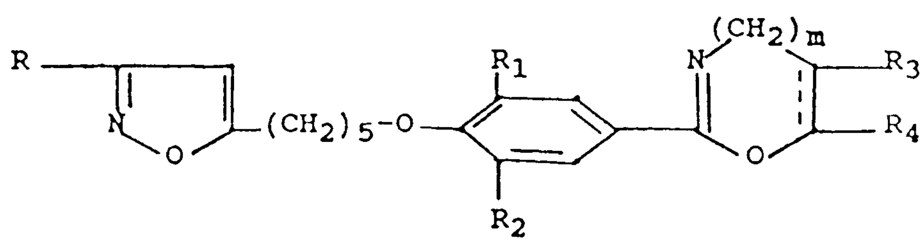

dans laquelle :

m est 0 ou 1,

R est méthyle ou hydroxyméthyle,

$R_1$ et $R_2$ sont chacun un halogène, méthyle, nitro ou trifluorométhyle, et

$R_3$ et $R_4$ sont chacun l'hydrogène ou un alkyle inférieur de 1 à 3 atomes de carbone, ou ses sels d'addition d'acide pharmaceutiquement acceptables.

3. Composé selon la revendication 2, qui est le 5-{5-[2,6-dichloro-4-(4,5-dihydro-2-oxazolyl)phénoxy]-pentyl}isoxazole ou un sel d'addition d'acide pharmaceutiquement acceptable de celui-ci.

4. Composé de formule :

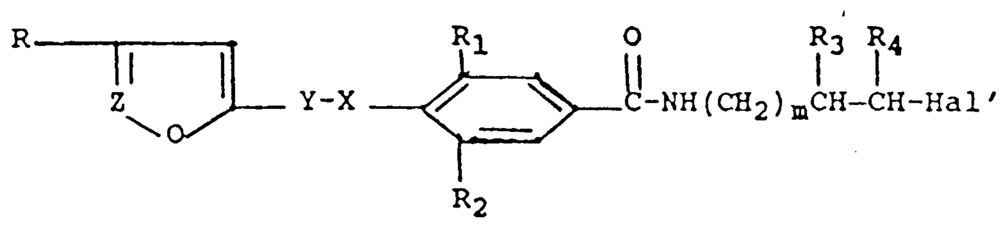

dans laquelle :

Hal' est le chlore ou le brome,

X est 0 ou $CH_2$,

Y est un pont alkylène de 3 à 9 atomes de carbone

Z est N ou $R_5C$, où $R_5$ est l'hydrogène ou un alcanoyle inférieur de 1 à 4 atomes de carbone,

m est 0 ou 1,

$R_1$ et $R_2$ sont des halogènes, méthyles, nitro, alcoxycarbonyles inférieurs de 1 à 4 atomes de carbone ou trifluorométhyles, et

R, $R_3$ et $R_4$ sont des hydrogènes ou des alkyles inférieurs de 1 à 3 atomes de carbone à condition que lorsque Z est N, R soit un alkyle inférieur de 1 à 4 atomes de carbone.

**5.** Composition pour combattre les virus qui comprend une quantité efficace du point de vue antiviral d'un composé selon l'une quelconque des revendications 1 à 4 en mélange avec un véhicule ou diluant approprié.

**6.** Procédé de préparation d'un composé de formule :

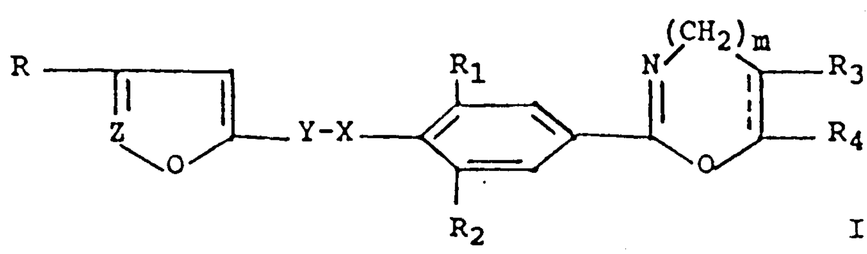

I

dans laquelle :

X est 0 ou $CH_2$,

Y est un pont alkylène de 3 à 9 atomes de carbone ou un chaînon oléfinique de 5 atomes de carbone comportant une liaison insaturée, éventuellement interrompu par un chaînon oléfinique,

Z est N ou $R_5C$ où $R_5$ est l'hydrogène ou un alcanoyle inférieur de 1 à 4 atomes de carbone à condition que lorsque Z est N, R soit un alkyle de 1 à 3 atomes de carbone,

m est 0 ou 1,

R est l'hydrogène ou un alkyle de 1 à 3 atomes de carbone éventuellement substitué par hydroxy, ou un alcoxy inférieur de 1 à 4 atomes de carbone,

$R_1$ et $R_2$ sont des halogènes, méthyles, nitro ou trifluorométhyles, et

$R_3$ et $R_4$ sont des hydrogènes ou des alkyles inférieurs de 1 à 3 atomes de carbone,

qui comprend

(a) pour préparer un composé sans double liaison en position 4, 5 du cycle oxazole ou 5,6-oxazine et dans lequel R est l'hydrogène ou un alkyle inférieur de 1 à 3 atomes de carbone, la cyclisation d'un composé de formule :

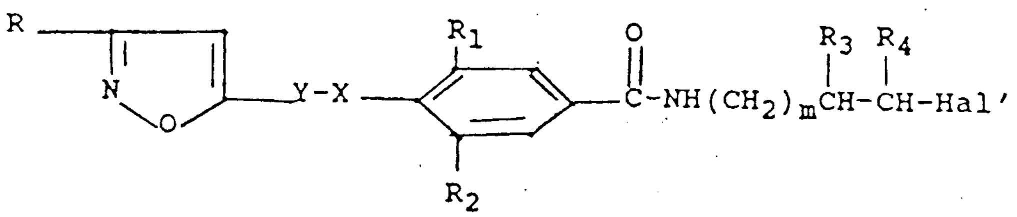

dans laquelle Hal' est le chlore ou le brome, par chauffage de ce dernier dans un solvant inerte en présence d'un accepteur d'acide, à l'exclusion des composés dans lesquels Y est un pont alkylène interrompu par un chaînon oléfinique, ou

(b) pour préparer un composé dans lequel X est O, la réaction d'un composé de formule :

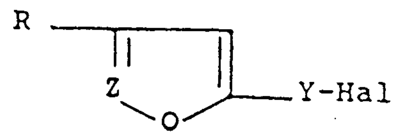

dans laquelle Hal est le chlore, le brome ou l'iode, avec un sel alcalin d'un composé de formule :

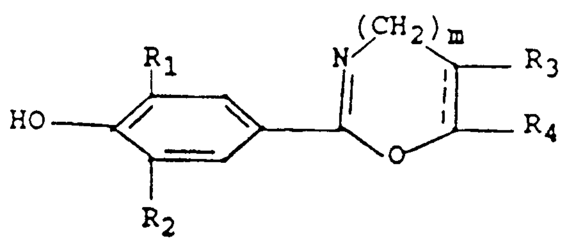

,

si on le souhaite, l'éthérification d'un composé de formule I dans lequel R est un hydroxyalkyle par réaction avec un halogénure d'alkyle inférieur en présence d'une base forte pour former un composé dans lequel R est un alcoxy inférieur alkyle inférieur, inférieur signifiant $C_1$-$C_4$

et, si on le souhaite, la conversion d'un composé basique obtenu en un sel d'addition d'acide de celui-ci.

**Revendications pour l'Etat contractant suivant : AT**

1. Procédé de préparation d'un composé de formule :

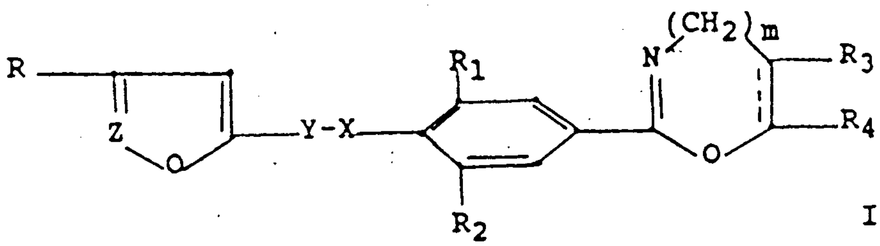

I

dans laquelle :

X est 0 ou $CH_2$,

Y est un pont alkylène de 3 à 9 atomes de carbone ou un chaînon oléfinique de 5 atomes de carbone avec une liaison insaturée,

Z est N ou $R_5C$, où $R_5$ est l'hydrogène ou un alcanoyle inférieur de 1 à 4 atomes de carbone, à condition que lorsque Z est N, R soit un alkyle de 1 à 3 atomes de carbone,

m est 0 ou 1,

R est l'hydrogène ou un alkyle de 1 à 3 atomes de carbone éventuellement substitué par hydroxy, ou un alcoxy inférieur de 1 à 4 atomes de carbone,

$R_1$ et $R_2$ sont des halogènes, méthyles, nitro ou trifluorométhyles, et

$R_3$ et $R_4$ sont des hydrogènes ou des alkyles inférieurs de 1 à 3 atomes de carbone, caractérisé en ce qu'il comprend :

(a) pour préparer un composé sans double liaison en position 4, 5 du cycle oxazole ou 5,6-oxazine et dans lequel R est l'hydrogène ou un alkyle inférieur de 1 à 3 atomes de carbone, la cyclisation d'un composé de formule :

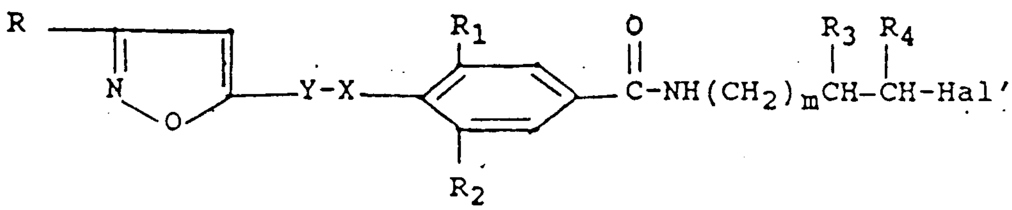

dans laquelle Hal' est le chlore ou le brome, par chauffage de ce dernier dans un solvant inerte en présence d'un accepteur d'acide, à l'exclusion des composés dans lesquels Y est un pont alkylène interrompu par un chaînon oléfinique, ou

(b) pour préparer un composé dans lequel X est 0, la réaction d'un composé de formule :

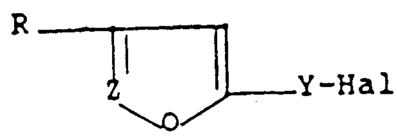

dans laquelle Hal est le chlore, le brome ou l'iode, avec un sel de métal alcalin d'un composé de formule :

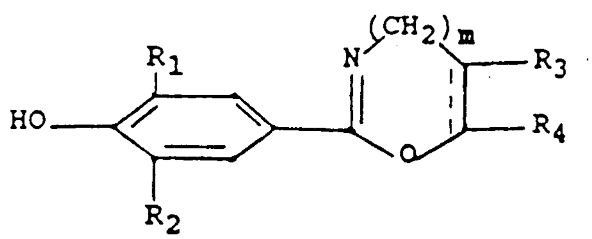

,

si on le souhaite, l'éthérification d'un composé de formule I dans lequel R est un hydroxyalkyle par réaction avec un halogénure d'alkyle inférieur de 1 à 4 atomes de carbone en présence d'une base forte pour former un composé dans lequel R est un alcoxyalkyle inférieur, respectivement,

et, si on le souhaite, la conversion d'un composé basique obtenu en un sel d'addition d'acide de celui-ci.

**2.** Procédé selon la revendication 1, caractérisé par la préparation d'un composé de formule :

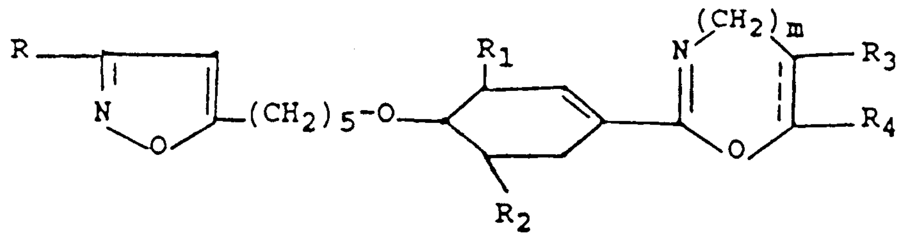

dans laquelle

m est 0 ou 1,

R est méthyle ou hydroxyméthyle,

$R_1$ et $R_2$ sont chacun un halogène, méthyle, nitro ou trifluorométhyle, et

$R_3$ et $R_4$ sont chacun l'hydrogène ou un alkyle inférieur de 1 à 3 atomes de carbone, ou de sels d'addition d'acide pharmaceutiquement acceptables de celui-ci.

**3.** Procédé selon la revendication 2, caractérisé par la préparation du 5-{5-[2,6-dichloro-4-(4,5-dihydro-2-oxazolyl)phénoxy]pentyl}isoxazole ou d'un sel d'addition d'acide pharmaceutiquement acceptable de celui-ci.